(19) **Europäisches Patentamt**
**European Patent Office**
**Office européen des brevets**

(11) **EP 2 979 607 A1**

(12) **EUROPEAN PATENT APPLICATION**
published in accordance with Art. 153(4) EPC

(43) Date of publication:
**03.02.2016 Bulletin 2016/05**

(51) Int Cl.:
**A61B 1/00** *(2006.01)*    **A61B 1/04** *(2006.01)*
**G02B 23/24** *(2006.01)*    **G06T 1/00** *(2006.01)*

(21) Application number: **13880591.6**

(22) Date of filing: **24.09.2013**

(86) International application number:
**PCT/JP2013/075629**

(87) International publication number:
**WO 2014/155778 (02.10.2014 Gazette 2014/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.03.2013 JP 2013067422**

(71) Applicant: **Olympus Corporation**
**Shibuya-ku**
**Tokyo 151-0072 (JP)**

(72) Inventor: **MORITA, Yasunori**
**Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **IMAGE PROCESSING DEVICE, ENDOSCOPIC DEVICE, PROGRAM AND IMAGE PROCESSING METHOD**

(57)    An image processing device includes an image acquisition section (305) that acquires a captured image in time series, the captured image including an image of the object, a distance information acquisition section (340) that acquires distance information based on the distance from an imaging section (200) to the object when the imaging section (200) captured the captured image, a motion detection section (380) that detects motion information about a local motion of the object based on the captured image acquired in time series, a classification section (310) that performs a classification process that classifies a structure of the object based on the distance information, and an enhancement processing section (330) that performs an enhancement process on the captured image based on results of the classification process, and controls the target or the enhancement level of the enhancement process corresponding to the motion information about the local motion of the object.

FIG. 3

**Description**

TECHNICAL FIELD

The present invention relates to an image processing device, an endoscope apparatus, a program, an image processing method, and the like.

BACKGROUND ART

A ductal structure (that is referred to as "pit pattern") observed on the surface of tissue may be used as an index when observing tissue, and making a diagnosis. For example, the pit pattern has been used to find (diagnose) an early lesion in the large intestine. This diagnostic method is referred to as "pit pattern diagnosis". The pit patterns are classified into six types (type I to type V) corresponding to the type of lesion, and the pit pattern diagnosis determines the type to which the observed pit pattern belongs.

Patent Document 1 discloses a device that acquires a three-dimensional optical tomographic image using an endoscope apparatus and an optical probe. Patent Document 1 discloses a method that samples XY plane images (that are perpendicular to the depth direction of tissue) at a plurality of depth positions based on the three-dimensional optical tomographic image, and enhances the pit pattern based on the average image.

RELATED-ART DOCUMENT

PATENT DOCUMENT

Patent Document 1: JP-A-2010-68865

SUMMARY OF THE INVENTION

TECHNICAL PROBLEM

For example, the structure of tissue within the captured image may be detected, and subjected to a classification process in order to implement the above enhancement process or the like. However, when implementing a method that detects a structure from the captured image, and subjects the detected structure to the classification process, a structure may be erroneously detected when a motion blur has occurred due to pulsation or the like since sufficient information may not be obtained from the image. For example, a matching process may be performed on a known pit pattern shape and the captured image when detecting the pit pattern (see above). However, it may be impossible to detect the pit pattern by the matching process when a motion blur has occurred, and incorrect classification results may be obtained.

Several aspects of the invention may provide an image processing device, an endoscope apparatus, a program, an image processing method, and the like that make it possible to suppress a situation in which a process is performed based on incorrect classification results.

SOLUTION TO PROBLEM

According to one aspect of the invention, there is provided an image processing device comprising:

an image acquisition section that acquires a captured image in time series, the captured image including an image of an object;
a distance information acquisition section that acquires distance information based on a distance from an imaging section to the object when the imaging section captured the captured image;
a motion detection section that detects motion information about a local motion of the object based on the captured image acquired in time series;
a classification section that performs a classification process that classifies a structure of the object based on the distance information; and
an enhancement processing section that performs an enhancement process on the captured image based on results of the classification process, and controls a target or an enhancement level of the enhancement process corresponding to the motion information about the local motion of the object.

According to one aspect of the invention, the classification process that classifies the structure of the object is performed based on the distance information, and the enhancement process is performed on the captured image based on the results of the classification process. The target or the enhancement level of the enhancement process is controlled corresponding to the motion information about the local motion of the object. This makes it possible to suppress a situation in which the enhancement process is performed based on incorrect classification results.

According to another aspect of the invention, there is provided an image processing device comprising:

an image acquisition section that acquires a captured image in time series, the captured image including an image of an object;

a distance information acquisition section that acquires distance information based on a distance from an imaging section to the object when the imaging section captured the captured image;

a motion detection section that detects motion information about a local motion of the object based on the captured image acquired in time series; and

a classification section that performs a classification process that classifies a structure of the object based on the distance information, and controls a target of the classification process corresponding to the motion information about the local motion of the object.

According to this aspect of the invention, the classification process that classifies the structure of the object is performed based on the distance information, and the target of the classification process is controlled corresponding to the motion information about the local motion of the object. This makes it possible to suppress a situation in which the subsequent process is performed based on incorrect classification results.

According to another aspect of the invention, there is provided an endoscope apparatus comprising one of the above image processing devices.

According to another aspect of the invention, there is provided a program that causes a computer to perform steps of:

acquiring a captured image in time series, the captured image including an image of an object;

acquiring distance information based on a distance from an imaging section to the object when the imaging section captured the captured image;

detecting motion information about a local motion of the object based on the captured image acquired in time series;

performing a classification process that classifies a structure of the object based on the distance information; and

performing an enhancement process on the captured image based on results of the classification process, and controlling a target or an enhancement level of the enhancement process corresponding to the motion information about the local motion of the object.

According to another aspect of the invention, there is provided a program that causes a computer to perform steps of:

acquiring a captured image in time series, the captured image including an image of an object;

acquiring distance information based on a distance from an imaging section to the object when the imaging section captured the captured image;

detecting motion information about a local motion of the object based on the captured image acquired in time series; and

performing a classification process that classifies a structure of the object based on the distance information, and controlling a target of the classification process corresponding to the motion information about the local motion of the object.

According to another aspect of the invention, there is provided an image processing method comprising:

acquiring a captured image in time series, the captured image including an image of an object;

acquiring distance information based on a distance from an imaging section to the object when the imaging section captured the captured image;

detecting motion information about a local motion of the object based on the captured image acquired in time series;

performing a classification process that classifies a structure of the object based on the distance information; and

performing an enhancement process on the captured image based on results of the classification process, and controlling a target or an enhancement level of the enhancement process corresponding to the motion information about the local motion of the object.

According to another aspect of the invention, there is provided an image processing method comprising:

acquiring a captured image in time series, the captured image including an image of an object;

acquiring distance information based on a distance from an imaging section to the object when the imaging section captured the captured image;

detecting motion information about a local motion of the object based on the captured image acquired in time series; and

performing a classification process that classifies a structure of the object based on the distance information, and controlling a target of the classification process corresponding to the motion information about the local motion of the object.

BRIEF DESCRIPTION OF DRAWINGS

FIG. 1A illustrates the relationship between an imaging section and the object when observing an abnormal area, and FIG. 1B illustrates an example of the acquired image.

FIG. 2A illustrates the relationship between an imaging section and the object when a motion blur has occurred, and FIG. 2B illustrates an example of the acquired image.

FIG. 3 illustrates a first configuration example of an image processing device.

FIG. 4 illustrates a second configuration example of an image processing device.

FIG. 5 illustrates a configuration example of an endoscope apparatus.

FIG. 6 illustrates a detailed configuration example of an image processing section (first embodiment).

FIG. 7 illustrates an example of an image that has not been subjected to a distortion correction process, and an image obtained by the distortion correction process.

FIG. 8 is a view illustrating a classification process.

FIG. 9 illustrates an example of a flowchart of a process performed by an image processing section.

FIG. 10 illustrates a detailed configuration example of an image processing section (second embodiment).

FIG. 11 illustrates a detailed configuration example of an image processing section (modification of second embodiment).

FIG. 12 illustrates a detailed configuration example of an image processing section (third embodiment).

FIG. 13 illustrates a detailed configuration example of an image processing section (fourth embodiment).

FIG. 14A illustrates the relationship between an imaging section and the object (fourth embodiment), and FIGS. 14B and 14C illustrate an example of the acquired image.

FIG. 15 illustrates an example of a table in which the magnification of an optical system is linked to distance.

FIG. 16 illustrates a detailed configuration example of a classification section.

FIGS. 17A and 17B are views illustrating a process performed by a surface shape calculation section.

FIG. 18A illustrates an example of a basic pit, and FIG. 18B illustrates an example of a corrected pit.

FIG. 19 illustrates a detailed configuration example of a surface shape calculation section.

FIG. 20 illustrates a detailed configuration example of a classification processing section when implementing a first classification method.

FIGS. 21A to 21F are views illustrating a specific example of a classification process.

FIG. 22 illustrates a detailed configuration example of a classification processing section when implementing a second classification method.

FIG. 23 illustrates an example of a classification type when a plurality of classification types are used.

FIGS. 24A to 24F illustrate an example of a pit pattern.

DESCRIPTION OF EMBODIMENTS

Exemplary embodiments of the invention are described below. Note that the following exemplary embodiments do not in any way limit the scope of the invention laid out in the claims. Note also that all of the elements described in connection with the following exemplary embodiments should not necessarily be taken as essential elements of the invention.

1. Outline

An outline of several embodiments of the invention is described below taking an example in which an endoscope apparatus performs a pit pattern classification process.

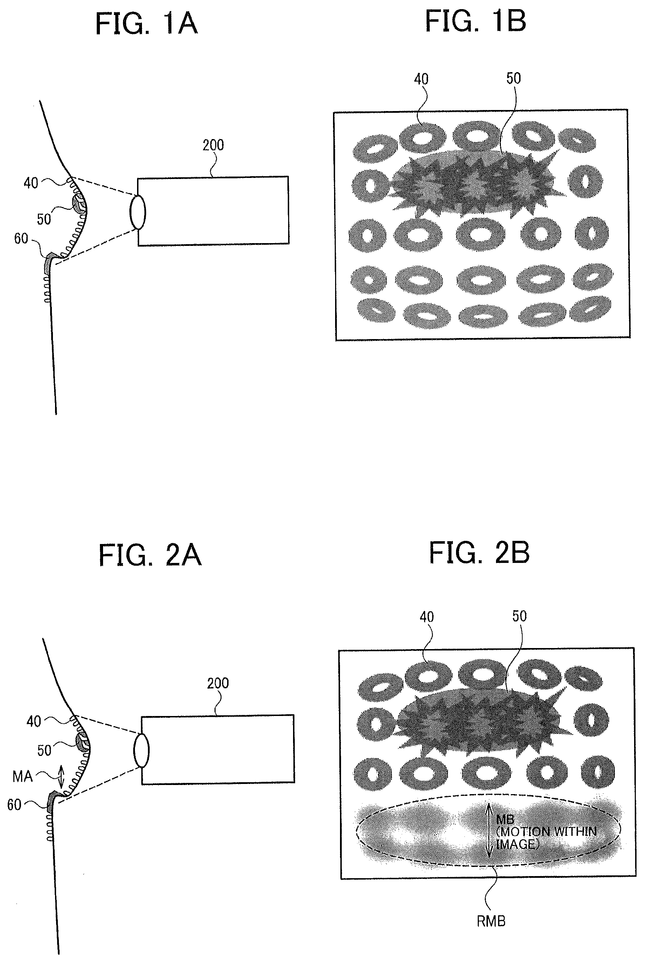

FIG. 1A illustrates the relationship between an imaging section 200 and the object when observing an abnormal part (e.g., early lesion). FIG. 1B illustrates an example of an image acquired when observing the abnormal part. A normal duct 40 represents a normal pit pattern, an abnormal duct 50 represents an abnormal pit pattern having an irregular shape, and a duct disappearance area 60 represents an abnormal area in which the pit pattern has disappeared due to a lesion.

When the operator (user) has found an abnormal part (abnormal duct 50 and duct disappearance area 60) (see FIG. 1A), the operator brings the imaging section 200 closer to the abnormal part so that the imaging section 200 directly faces the abnormal part as much as possible. As illustrated in FIG. 1B, a normal part (normal duct 40) has a pit pattern in which regular structures are uniformly arranged.

Such a normal part can be detected by way of image processing by registering or learning a normal pit pattern structure in advance as known characteristic information (prior information), and performing a matching process, for example. On the other hand, the pit pattern has an irregular shape (or has disappeared) in an abnormal part (i.e., the pit pattern in an abnormal part has various shapes as compared with a normal part). Therefore, it is difficult to detect an abnormal part based on the known characteristic information. According to several embodiments of the invention, an area that has not been detected as a normal part is classified as an abnormal part (i.e., the pit patterns are classified into a normal part and an abnormal part). It is possible to prevent a situation in which an abnormal part is missed, and improve the accuracy of qualitative diagnosis by enhancing an abnormal part that has been detected (classified) in this manner.

According to the above method, however, since an area that has not been detected as a normal part is detected as an abnormal part, an area other than an early lesion may also be detected as an abnormal part (i.e., erroneous detection may occur). For example, when the object makes a motion (moves) relative to the imaging section due to pulsation or the like, the image of the object within the captured image may be blurred due to a motion blur, and erroneous detection may occur due to the motion blur. Note that the term "motion blur" used herein refers to a state in which part or the entirety of the image is blurred due to the motion (movement) of the object or the imaging section.

FIG. 2A illustrates the relationship between the imaging section 200 and the object when a motion blur has occurred. FIG. 2B illustrates an example of an image acquired when a motion blur has occurred. When part of tissue has made a motion MA (see FIG. 2A), a motion blur MB occurs within the image (see the lower part of the image illustrated in FIG. 2B). Since the structure of the object cannot be clearly observed (determined) in an area RMB in which the motion blur MB has occurred, the area RMB is not detected as a normal part by the matching process, and is classified as an abnormal part. The area RMB is an area that should be displayed as a normal part, but is displayed as an abnormal part since the area RMB has been classified as an abnormal part.

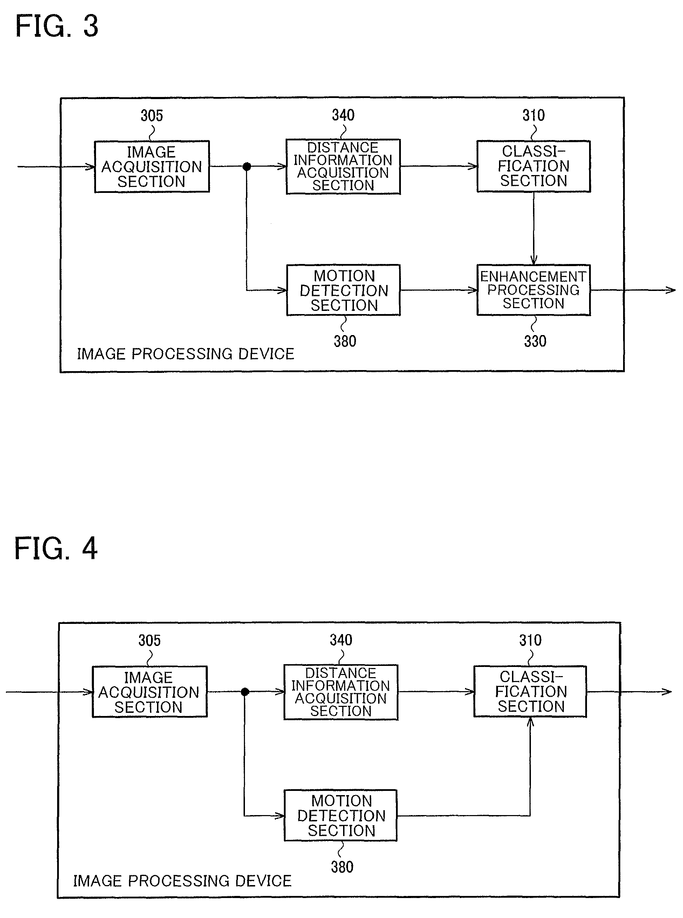

An image processing device according to several embodiments of the invention includes an image acquisition section 305 that acquires a captured image in time series, the captured image including an image of the object, a distance information acquisition section 340 that acquires distance information based on the distance from the imaging section 200 to the object when the imaging section 200 captured the captured image, a motion detection section 380 that detects motion information about a local motion of the object based on the captured image acquired in time series, a classification section 310 that performs a classification process that classifies the structure of the object based on the distance information, and an enhancement processing section 330 that performs an enhancement process on the captured image based on the results of the classification process, and controls the target or the enhancement level of the enhancement process corresponding to the motion information about the local motion of the object (see FIG. 3).

According to this configuration, the area RMB within the image for which the reliability of the classification results decreases due to a motion blur can be detected by detecting the motion information about the local motion of the object. It is possible to suppress a situation in which the area RMB is enhanced based on the results of the classification process having low reliability by controlling the target or the enhancement level of the enhancement process corresponding to the motion information about the local motion of the object.

For example, the classification process based on the distance information calculates the shape of the surface of the object from the distance information, performs a matching process on a reference pit pattern (that has been deformed corresponding to the shape of the surface of the object) and the image, and classifies the pit pattern within the image based on the matching results. In this case, the accuracy of the matching process decreases when a motion blur has occurred. According to several embodiments of the invention, however, it is possible to prevent erroneous display due to a decrease in the accuracy of the matching process.

Since the object is normally observed in a state in which the imaging section 200 is brought close to the object when performing pit pattern diagnosis, a significant motion blur occurs within the image even when the object has made only a small motion. Therefore, erroneous detection can be effectively suppressed by detecting an abnormal part during pit pattern diagnosis while excluding the effects of a motion blur.

The image processing device according to several embodiments of the invention may include the image acquisition section 305 that acquires a captured image in time series, the captured image including an image of the object, the distance information acquisition section 340 that acquires the distance information based on the distance from the imaging section 200 to the object when the imaging section 200 captured the captured image, the motion detection section 380 that detects the motion information about the local motion of the object based on the captured image acquired in time series, and the classification section 310 that performs the classification process that classifies the structure of the object based on the distance information, and controls the target of the classification process corresponding to the motion information about the local motion of the object (see FIG. 4).

According to this configuration, a situation in which incorrect classification results are obtained for the area RMB within the image for which the reliability of the classification results decreases due to a motion blur can be suppressed by controlling the target of the classification process corresponding to the motion information about the local motion of the object. When employing the configuration illustrated in FIG. 4, the results of the classification process may be used for information processing other than the enhancement process, or may be output to an external device, and used for a process performed by the external device. It is possible to improve the reliability of the processing results of these processes by suppressing a situation in which incorrect classification results are obtained.

The term "distance information" used herein refers to information that links each position of the captured image to the distance to the object at each position of the captured image. For example, the distance information is a distance map in which the distance to the object in the optical axis direction of the imaging section 200 is linked to each pixel. Note that the distance information is not limited to the distance map, but may be various types of information that are acquired based on the distance from the imaging section 200 to the object (described later).

The classification process is not limited to a pit pattern classification process. The term "classification process" used herein refers to an arbitrary process that classifies the structure of the object corresponding to the type, the state, or the like of the structure. The term "structure" used herein in connection with the object refers to a structure that can assist the user in observation and diagnosis when the classification results are presented to the user. For example, when the endoscope apparatus is a medical endoscope apparatus, the structure may be a pit pattern, a polyp that projects from a mucous membrane, the folds of the digestive tract, a blood vessel, or a lesion (e.g., cancer). The classification process classifies the structure of the object corresponding to the type, the state (e.g., normal/abnormal), or the degree of abnormality of the structure.

Note that the classification process based on the distance information is not limited to the pit pattern classification process described above. Various other classification processes may also be used. For example, a stereo matching process is performed on the stereo image to acquire a distance map, and a low-pass filtering process, a morphological process, or the like is performed on the distance map to acquire global shape information about the object. The global shape information is subtracted from the distance map to acquire information about a local concave-convex structure. The known characteristic information (e.g., the size and the shape of a specific polyp, or the depth and the width of a groove specific to a lesion) about the classification target structure is compared with the information about a local concave-convex structure to extract a concave-convex structure that agrees with the known characteristic information. A specific structure (e.g., polyp or groove) can thus be classified (detected). In this case, the accuracy of the stereo matching process may decrease due to a motion blur, and incorrect distance information may be acquired. The classification accuracy decreases if a concave-convex structure is classified based on the incorrect distance information. According to several embodiments of the invention, however, it is possible to prevent erroneous display due to such a decrease in accuracy.

The term "enhancement process" used herein refers to a process that enhances or differentiates a specific target within the image. For example, the enhancement process may be a process that enhances the structure, the color, or the like of an area that has been classified as a specific type or a specific state, or may be a process that highlights such an area, or may be a process that encloses such an area with a line, or may be a process that adds a mark that represents such an area. A specific area may be caused to stand out (or differentiated) by performing the above process on an area other than the specific area.

2. First embodiment

2.1. Endoscope apparatus

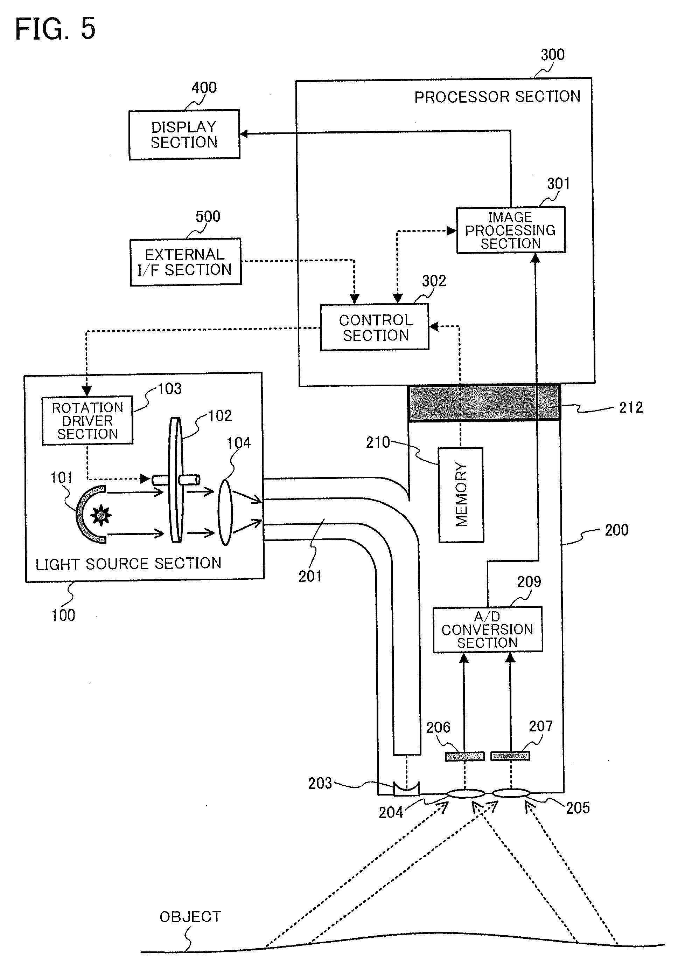

A detailed configuration according to a first embodiment of the invention is described below. FIG. 5 illustrates a configuration example of an endoscope apparatus. The endoscope apparatus includes a light source section 100, an imaging section 200, a processor section 300 (control device), a display section 400, and an external I/F section 500.

The light source section 100 includes a white light source 101, a rotary color filter 102 that includes a plurality of color filters that differ in spectral transmittance, a rotation driver section 103 that drives the rotary color filter 102, and a condenser lens 104 that focuses light (that has passed through the rotary color filter 102 and has spectral characteristics) on the incident end face of a light guide fiber 201.

The rotary color filter 102 includes a red color filter, a green color filter, a blue color filter, and a rotary motor.

The rotary driver section 103 rotates the rotary color filter 102 at a given rotational speed in synchronization with the imaging period of an image sensor 206 and an image sensor 207 based on a control signal output from a control section 302 included in the processor section 300. For example, when the rotary color filter 102 is rotated at 20 revolutions per second, each color filter crosses the incident white light every 1/60th of a second. In this case, the image sensor 206 and the image sensor 207 capture the reflected light from the observation target to which each color light (R, G, or B) has been applied, and transfer the resulting image every 1/60th of a second. Specifically, the endoscope apparatus according to the first embodiment frame-sequentially captures an R image, a G image, and a B image every 1/60th of a second, and the substantial frame rate is 20 fps.

The imaging section 200 is formed to be elongated and flexible so that the imaging section 200 can be inserted into a body cavity (e.g., stomach or large intestine), for example. The imaging section 200 includes the light guide fiber 201 that guides the light focused by the light source section 100, and an illumination lens 203 that diffuses the light guided by the light guide fiber 201 to illuminate the observation target. The imaging section 200 also includes an objective lens 204 and an objective lens 205 that focus the reflected light from the observation target, the image sensor 206 and the image sensor 207 that detect the focused light, and an A/D conversion section 209 that converts (photoelectrically converted) analog signals output from the image sensor 206 and the image sensor 207 into digital signals. The imaging section 200 further includes a memory 210 that stores scope ID information and specific information (including production variations) about the imaging section 200, and a connector 212 that is removably connected to the processor section 300. The image sensor 206 and the image sensor 207 are monochrome single-chip image sensors, for example. A CCD image sensor, a CMOS image sensor, or the like may be used as the image sensor 206 and the image sensor 207.

The objective lens 204 and the objective lens 205 are disposed at a given interval so that a given parallax image (hereinafter referred to as "stereo image") can be captured. The objective lens 204 and the objective lens 205 respectively form a left image and a right image on the image sensor 206 and the image sensor 207. The A/D conversion section 209 converts the left image output from the image sensor 206 and the right image output from the image sensor 207 into digital signals, and outputs the resulting left image and the resulting right image to an image processing section 301. The memory 210 is connected to the control section 302, and transmits the scope ID information and the specific information (including production variations) to the control section 302.

The processor section 300 includes the image processing section 301 (corresponding to an image processing device) that performs various types of image processing on the image transmitted from the A/D conversion section 209, and the control section 302 that controls each section of the endoscope apparatus.

The display section 400 displays the image transmitted from the image processing section 301. The display section 400 is a display device (e.g., CRT or liquid crystal monitor) that can display a moving image (movie (video)).

The external I/F section 500 is an interface that allows the user to input information and the like to the endoscope apparatus. For example, the external I/F section 500 includes a power switch (power ON/OFF switch), a shutter button (capture start button), a mode (e.g., imaging mode) switch (e.g., a switch for selectively enhancing the structure of the surface of tissue), and the like. The external I/F section 500 outputs the input information to the control section 302.

2.2. Image processing section

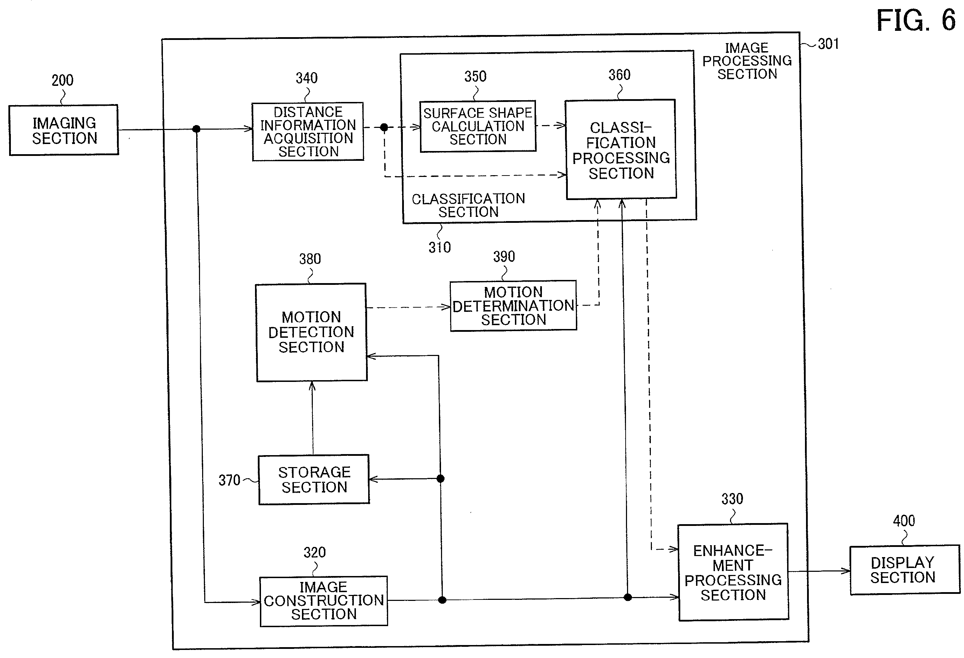

FIG. 6 illustrates a detailed configuration example of the image processing section 301 according to the first embodiment. The image processing section 301 includes a classification section 310, an image construction section 320, an enhancement processing section 330, a distance information acquisition section 340 (distance map calculation section), a storage section 370, a motion detection section 380, and a motion determination section 390. Although an example in which the pit pattern classification process is performed by utilizing the matching process is described below, various other classification processes that utilize the distance information may also be used.

The imaging section 200 is connected to the image construction section 320 and the distance information acquisition section 340. A classification processing section 360 is connected to the enhancement processing section 330. The image construction section 320 is connected to the classification processing section 360, the enhancement processing section 330, the storage section 370, and the motion detection section 380. The enhancement processing section 330 is connected to the display section 400. The distance information acquisition section 340 is connected to the classification processing section 360 and a surface shape calculation section 350. The surface shape calculation section 350 is connected to the classification processing section 360. The storage section 370 is connected to the motion detection section 380. The motion detection section 380 is connected to the motion determination section 390. The motion determination section 390 is connected to the classification processing section 360. The control section 302 (not illustrated in FIG. 6) is bidirectionally connected to each section of the image processing section 301, and controls each section of the image processing section 301.

The distance information acquisition section 340 acquires the stereo image output from the A/D conversion section 209, and acquires the distance information based on the stereo image. Specifically, the distance information acquisition section 340 performs a matching calculation process on the left image (reference image) and a local area of the right image along an epipolar line that passes through the attention pixel (pixel in question) situated at the center of a local area of the left image to calculate a position at which the maximum correlation is obtained as a parallax. The distance information acquisition section 340 converts the calculated parallax into the distance in the Z-axis direction to acquire the distance information, and outputs the distance information to the classification section 310.

The term "distance information" used herein refers to various types of information that are acquired based on the distance from the imaging section 200 to the object. For example, when implementing triangulation using a stereo optical system, the distance with respect to an arbitrary point of a plane that connects two lenses that produce a parallax may be used as the distance information. Alternatively, the distance information may be acquired using a Time-of-Flight method. When using a Time-of-Flight method, a laser beam or the like is applied to the object, and the distance is measured based on the time of arrival of the reflected light. In this case, the distance with respect to the position of each pixel of the plane of the image sensor that captures the reflected light may be acquired as the distance information, for example. Although an example in which the distance measurement reference point is set to the imaging section 200 has been described above, the reference point may be set at an arbitrary position other than the imaging section 200. For example, the reference point may be set at an arbitrary position within a three-dimensional space that includes the imaging section 200 and the object. The distance information acquired using such a reference point is also included within the scope of the term "distance information".

The distance from the imaging section 200 to the object may be the distance from the imaging section 200 to the object in the depth direction, for example. For example, the distance from the imaging section 200 to the object in the direction of the optical axis of the imaging section 200 may be used. Specifically, the distance to a given point of the object is the distance from the imaging section 200 to the object along a line that passes through the given point and is parallel to the optical axis. Examples of the distance information include a distance map. The term "distance map" used herein refers to a map in which the distance (depth) to the object in the Z-axis direction (i.e., the direction of the optical axis of the imaging section 200) is specified for each point in the XY plane (e.g., each pixel of the captured image), for example.

The distance information acquisition section 340 may set a virtual reference point at a position that can maintain a relationship similar to the relationship between the distance values of the pixels on the distance map acquired when the reference point is set to the imaging section 200, to acquire the distance information based on the distance from the imaging section 200 to each corresponding point. For example, when the actual distances from the imaging section 200 to three corresponding points are respectively "3", "4", and "5", the distance information acquisition section 340 may acquire distance information "1.5", "2", and "2.5" respectively obtained by halving the actual distances "3", "4", and "5" while maintaining the relationship between the distance values of the pixels.

The image construction section 320 acquires the stereo image (left image and right image) output from the A/D conversion section 209, and performs image processing (e.g., OB process, gain process, and y process) on the stereo image to generate an image that can be output from (displayed on) the display section 400. The image construction section 320 outputs the resulting image to the storage section 370, the motion detection section 380, the classification section 310, and the enhancement processing section 330.

The storage section 370 stores the time-series image transmitted from the image construction section 320. The storage section 370 stores images in a number equal to the number of images required for the motion detection process. For example, when comparing images that correspond to two frames to acquire a motion vector, the storage section 370 stores an image that corresponds to one frame.

The motion detection section 380 detects motion information about the object within the image based on the captured image. Specifically, the motion detection section 380 performs an optical system distortion correction process on the image that has been input from image construction section 320 and the image in the preceding frame that is stored in the storage section 370. The motion detection section 380 performs a feature point matching process on the images subjected to the distortion correction process, and calculates the motion amount corresponding to each pixel (or each area) from the motion vector of the feature point.

Various types of information that represents the motion of the object may be used as the motion information. For example, the motion vector that includes information about the magnitude and the direction of the motion may be used as the motion information, or only the magnitude (motion amount) of the motion vector may be used as the motion information. The inter-frame motion information may be averaged over a plurality of frames, and may be used as the motion information.

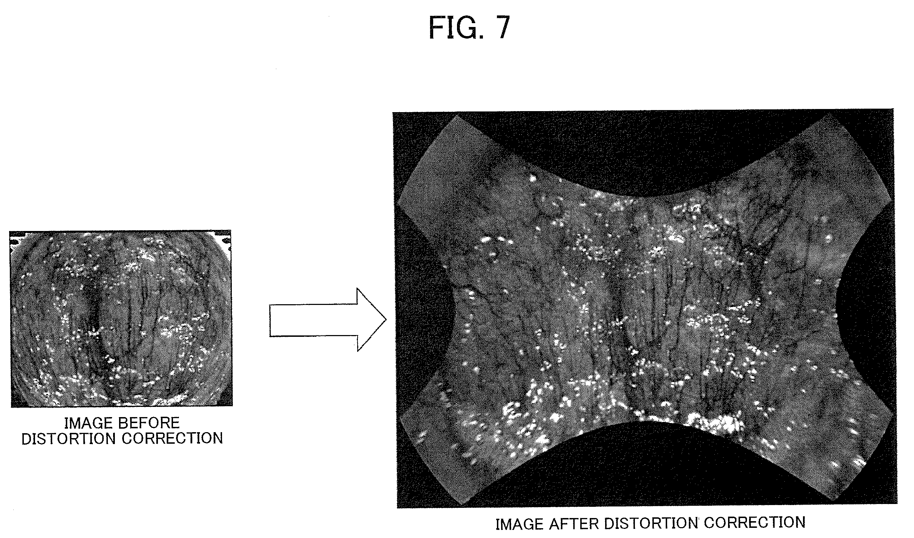

The distortion correction process corrects distortion (i.e., aberration). FIG. 7 illustrates an example of an image that has not been subjected to the distortion correction process, and an image obtained by the distortion correction process. The motion detection section 380 acquires the pixel coordinates of the image obtained by the distortion correction process. The size of the image obtained by the distortion correction process is acquired in advance based on the distortion of the optical system. The motion detection section 380 transforms the acquired pixel coordinates (x, y) into coordinates (x', y') around the optical center (i.e., origin) using the following expression (1). Note that (center_x, center_y) are the coordinates of the optical center after the distortion correction process. For example, the optical center after the distortion correction process is the center of the image obtained by the distortion correction process.

$$\begin{pmatrix} x' \\ y' \end{pmatrix} = \begin{pmatrix} x \\ y \end{pmatrix} - \begin{pmatrix} center\_x \\ center\_y \end{pmatrix} \qquad (1)$$

The motion detection section 380 calculates the object height r using the following expression (2) based on the pixel coordinates (x', y'). Note that max_r is the maximum object height within the image obtained by the distortion correction process.

$$r = \left( x'^2 + y'^2 \right)^{1/2} / \max\_r \qquad (2)$$

The motion detection section 380 calculates the ratio (R/r) of the image height to the object height based on the calculated object height r. Specifically, the relationship between the ratio R/r and the object height r is stored as a table, and the ratio R/r that corresponds to the object height r is acquired referring to the table.

The motion detection section 380 then acquires the pixel coordinates (X, Y) before the distortion correction process that corresponds to the pixel coordinates (x, y) after the distortion correction process using the following expression (3). Note that (center_X, center_Y) are the coordinates of the optical center before the distortion correction process. For example, the optical center before the distortion correction process is the center of the image that has not been subjected to the distortion correction process.

$$\begin{pmatrix} X \\ Y \end{pmatrix} = (R/r) \cdot \begin{pmatrix} x' \\ y' \end{pmatrix} + \begin{pmatrix} center\_X \\ center\_Y \end{pmatrix} \qquad (3)$$

The motion detection section 380 then calculates the pixel value at the pixel coordinates (x, y) after the distortion correction process based on the calculated pixel coordinates (X, Y) before the distortion correction process. When the pixel coordinates (X, Y) are not integers, the pixel value is calculated by performing a linear interpolation process based on the pixel values of the peripheral pixels. The motion detection section 380 performs the above process on each pixel of the image obtained by the distortion correction process. It is possible to accurately detect the motion amount corresponding to the center and the peripheral area of the image by performing the above distortion correction process.

The motion detection section 380 detects the motion amount corresponding to each pixel of the image obtained by the distortion correction process. Note that the motion amount at the coordinates (x', y') is represented by Mv(x', y'). The motion detection section 380 performs an inverse distortion correction process on the detected motion amount Mv(x', y') to convert the motion amount Mv(x', y') into the motion amount Mv(x, y) at the pixel position (x, y) before the distortion correction process. The motion detection section 380 transmits the motion amount Mv(x, y) to the motion determination section 390 as the motion information.

Although an example in which the motion amount is detected on a pixel basis has been described above, the configuration is not limited thereto. For example, the image may be divided into a plurality of local areas, and the motion amount may be detected on a local area basis. Although an example in which each process is performed on a pixel basis is described below, each process may be performed on a local area basis.

The motion determination section 390 determines whether or not the motion amount is large corresponding to each pixel of the image based on the motion information. Specifically, the motion determination section 390 detects a pixel for which the motion amount Mv(x, y) input from the motion detection section 380 is equal to or larger than a threshold value. The threshold value is set in advance corresponding to the number of pixels of the image, for example. Alternatively, the user may set the threshold value through the external I/F section 500. The motion determination section 390 transmits the determination results to the classification section 310.

The classification section 310 performs the classification process on the pixels of the image that correspond to the image of the structure based on the distance information and a classification reference. More specifically, the classification section 310 includes the surface shape calculation section 350 (three-dimensional shape calculation section) and the classification processing section 360. Note that the details of the classification process performed by the classification section 310 are described later. An outline of the classification process is described below.

The surface shape calculation section 350 calculates a normal vector to the surface of the object corresponding to each pixel of the distance map as surface shape information (three-dimensional shape information in a broad sense). The classification processing section 360 projects a reference pit pattern onto the surface of the object based on the normal vector. The classification processing section 360 adjusts the size of the reference pit pattern to the size within the image (i.e., an apparent size that decreases within the image as the distance increases) based on the distance at the corresponding pixel position. The classification processing section 360 performs a matching process on the corrected reference pit pattern and the image to detect an area that agrees with the reference pit pattern.

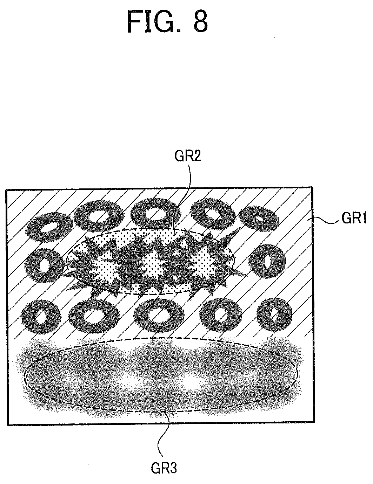

As illustrated in FIG. 8, the classification processing section 360 uses the shape of a normal pit pattern as the reference pit pattern, classifies an area GR1 that agrees with the reference pit pattern as a "normal part", and classifies an area GR2 that does not agree with the reference pit pattern as an "abnormal part (non-normal part)", for example. The classification processing section 360 classifies an area GR3 for which the motion determination section 390 has determined that the motion amount is equal to or larger than the threshold value as "unknown". Specifically, the classification processing section 360 excludes a pixel for which the motion amount is equal to or larger than the threshold value from the target of the matching process (i.e., classifies a pixel for which the motion amount is equal to or larger than the threshold value as "unknown"), and performs the matching process on the remaining pixels to classify these pixels as "normal part" or "abnormal part".

Note that the category "unknown" means that whether the structure belongs to "normal part" or "abnormal part" cannot be determined by the classification process that classifies the structure of the object corresponding to the type, the state (e.g., normal/abnormal), or the degree of abnormality of the structure. For example, when the structure of the object is classified as "normal part" or "abnormal part", the structure of the object that cannot be determined (that is not determined) to belong to "normal part" or "abnormal part" is classified as "unknown".

The enhancement processing section 330 performs the enhancement process on the image based on the results of the classification process. For example, the enhancement processing section 330 performs a filtering process or a color enhancement process that enhances the structure of the pit pattern on the area GR2 that has been classified as "abnormal part", and performs a process that applies a specific color that represents the category "unknown" on the area GR3 that has been classified as "unknown".

According to the first embodiment, it is possible to suppress a situation in which an abnormal part is erroneously detected even when a motion blur has occurred due to the motion (movement) of the object. Specifically, since the area GR3 of the image in which the motion amount of the object is large is excluded from the target of the (normal/abnormal) classification process, the area GR3 is not classified as "normal part" or "abnormal part". Therefore, since the enhancement process based on the (normal/abnormal) classification results is not performed on an area in which the image is blurred, it is possible to prevent a situation in which enhancement (enhancement display) is erroneously performed due to erroneous classification.

Since the motion amount is detected on a pixel basis (or a local area basis), it is possible to suppress a situation in which an abnormal part is erroneously detected in an area in which the motion amount is large, and accurately detect an abnormal part in an area in which the motion amount is small, even when a local motion blur has occurred.

Although an example in which the detection range of the classification process is set based on the motion information on a pixel basis (or a local area basis) has been described above, the configuration is not limited thereto. For example, whether or not to perform the classification process may be determined (set) corresponding to the entire image using the average of the motion information corresponding to each pixel as the motion information corresponding to the entire image. Alternatively, the motion amount may be used as an index of the classification process. Specifically, a configuration may be employed in which a pixel for which it has been determined that the motion amount is large is not determined to be "abnormal part".

2.3. Software

Although an example in which each section included in the processor section 300 is implemented by hardware has been described above, the configuration is not limited thereto. For example, a CPU may perform the process of each section on an image acquired using an imaging device and the distance information. Specifically, the process of each section may be implemented by software by causing the CPU to execute a program. Alternatively, part of the process of each section may be implemented by software.

In this case, a program stored in an information storage medium is read, and executed by a processor (e.g., CPU). The information storage medium (computer-readable medium) stores a program, data, and the like. The information storage medium may be an arbitrary recording medium that records (stores) a program that can be read by a computer system, such as a portable physical medium (e.g., CD-ROM, USB memory, MO disk, DVD disk, flexible disk (FD), magnetooptical disk, or IC card), a stationary physical medium (e.g., HDD, RAM, or ROM) that is provided inside or outside a computer system, or a communication medium that temporarily stores a program during transmission (e.g., a public line connected through a modem, or a local area network or a wide area network to which another computer system or a server is connected).

Specifically, a program is recorded on the recording medium so that the program can be read by a computer. A computer system (i.e., a device that includes an operation section, a processing section, a storage section, and an output section) implements an image processing device by reading the program from the recording medium, and executing the program. Note that the program need not necessarily be executed by a computer system. The embodiments of the invention may similarly be applied to the case where another computer system or a server executes the program, or another computer system and a server execute the program in cooperation.

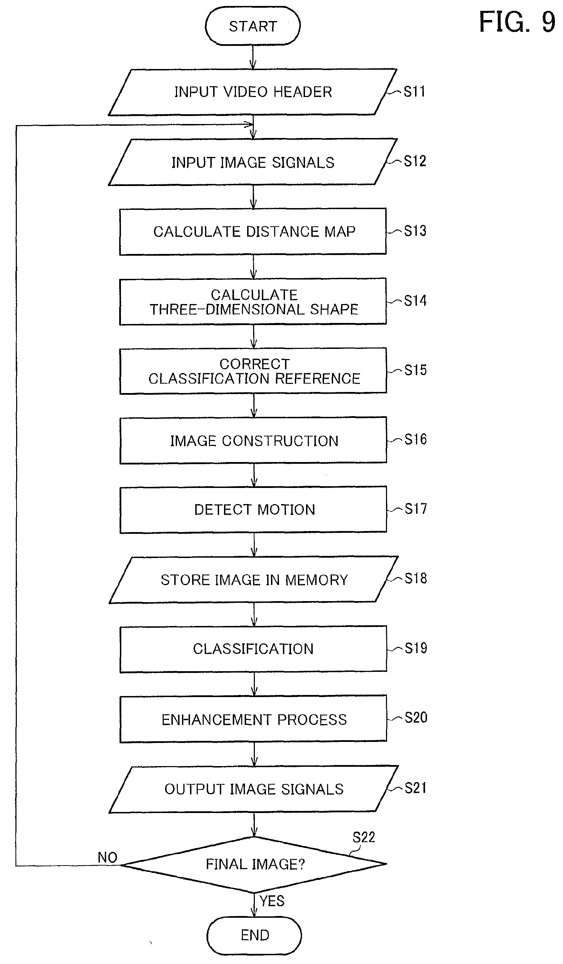

FIG. 9 is a flowchart when the process performed by the image processing section 301 is implemented by software.

As illustrated in FIG. 9, header information (e.g., the imaging conditions including the optical magnification (corresponding to the distance information) of the imaging device) is input (step S11). The stereo image signals (stereo image) captured by two image sensors are input (step S12). The distance map is calculated from the stereo image (step S 13). The surface shape (three-dimensional shape in a broad sense) is calculated from the distance map (step S 14). The classification reference (reference pit pattern) is corrected corresponding to the surface shape (step S15). The image construction process is then performed (step S16). The motion information is detected from the image obtained by the image construction process and the image in the preceding frame that is stored in the memory (step S 17). The image obtained by the image construction process (corresponding to one frame) is stored in the memory (step S18). An area in which the motion amount is small (i.e., an area in which a motion blur is not observed) is classified as "normal part" or "abnormal part" based on the motion information (step S19). The enhancement process is performed on an area that has been classified as "abnormal part" (step S20). The image subjected to the enhancement process is output (S21). The process is terminated when the final image of the movie has been processed. The step S12 is performed again when the final image of the movie has not been processed.

According to the first embodiment, the motion determination section 390 determines whether or not the motion amount of the object within each pixel or each area within the captured image is larger than the threshold value based on the motion information. The classification section 310 excludes the pixel or the area for which it has been determined that the motion amount is larger than the threshold value from the target of the classification process.

According to this configuration, an area of the image in which the motion amount of the object is large can be excluded from the target of the classification process. This makes it possible to suppress a situation in which the object is erroneously classified as a category that differs from the actual state of the object in an area in which the image is blurred due to a motion blur, and present correct information to the user to assist the user in making a diagnosis. Since the matching process is not performed on the pixel or the area for which it has been determined that the motion amount is large, the processing load can be reduced.

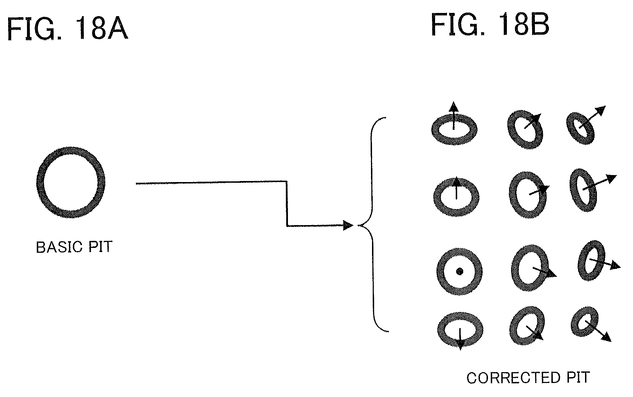

More specifically, the classification section 310 determines whether or not each pixel or each area agrees with the characteristics of a normal structure (e.g., the basic pit described later with reference to FIG. 18A) to classify each pixel or each area as a normal part or a non-normal part (abnormal part). The classification section 310 excludes the pixel or the area for which it has been determined that the motion amount is larger than the threshold value from the target of the process that classifies each pixel or each area as the normal part or the non-normal part, and classifies the pixel or the area for which it has been determined that the motion amount is larger than the threshold value as an unknown state that represents that it is unknown whether the pixel or the area should be classified as the normal part or the non-normal part.

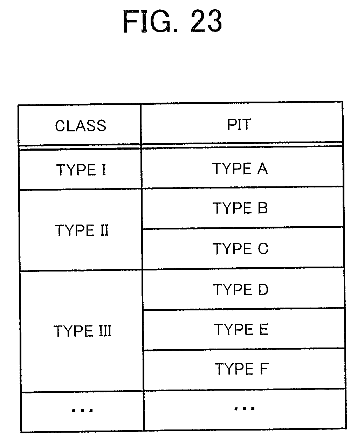

This makes it possible to classify the object as the normal part (e.g., a part in which a normal pit pattern is present) or the non-normal part other than the normal part, and suppress a situation in which the object in which a normal pit pattern is present is erroneously classified as the non-normal part in an area in which the image is blurred due to a motion blur. Note that the non-normal part may be classified into subcategories as described later with reference to FIG. 23 and the like. In such a case, a situation may also occur in which the object is erroneously classified due to a motion blur. According to the first embodiment, however, it is possible to suppress such a situation.

The classification section 310 may correct the result of the classification process with respect to the pixel or the area for which it has been determined that the motion amount is larger than the threshold value. Specifically, the classification section 310 may perform the (normal/non-normal) classification process independently of the results of the motion determination process, and then determine the final classification results based on the results of the motion determination process.

In this case, since the classification results in which the object is erroneously classified due to a motion blur are not output to the user, it is possible to present correct information to the user. For example, it is possible to notify the user of an area for which classification is impossible due to a large motion amount by correcting the classification result for the area to "unknown (unknown state)".

3. Second embodiment

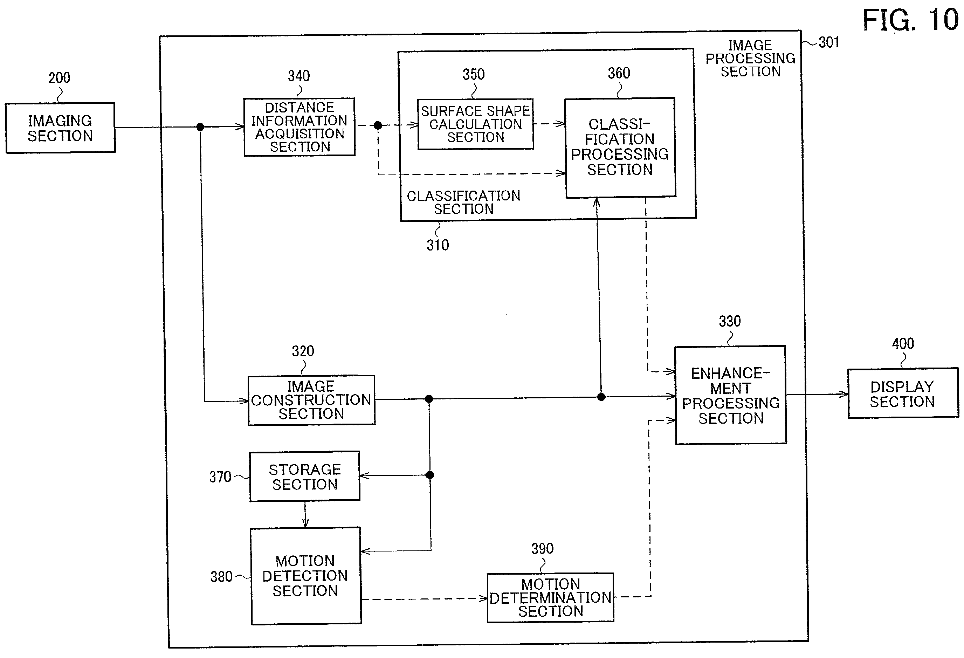

FIG. 10 illustrates a configuration example of an image processing section 301 according to a second embodiment. The image processing section 301 includes a classification section 310, an image construction section 320, an enhancement processing section 330, a distance information acquisition section 340, a storage section 370, a motion detection section 380, and a motion determination section 390. Note that the same elements as those described above in connection with the first embodiment are indicated by the same reference signs (symbols), and description thereof is appropriately omitted.

In the second embodiment, the motion determination section 390 is connected to the enhancement processing section 330. The classification section 310 classifies each pixel as "normal part" or "abnormal part" without performing the classification process based on the motion information. The enhancement processing section 330 controls the target of the enhancement process based on the determination results input from the motion determination section 390. Specifically, the enhancement processing section 330 does not perform the enhancement process on a pixel for which it has been determined that the motion amount is larger than the threshold value since the classification accuracy ("normal part" or "abnormal part") is low. Alternatively, the enhancement processing section 330 may perform the enhancement process that applies a specific color to a pixel for which it has been determined that the motion amount is larger than the threshold value to notify the user that the classification accuracy is low, for example.

According to the second embodiment, the motion determination section 390 determines whether or not the motion amount of the object within each pixel or each area within the captured image is larger than the threshold value based on the motion information. The enhancement processing section 330 excludes the pixel or the area for which it has been determined that the motion amount is larger than the threshold value from the target of the enhancement process based on the classification results.

According to this configuration, since the enhancement process based on the classification results is not performed in an area of the image in which the motion amount of the object is large, it is possible to present highly reliable classification results to the user even when the object has been erroneously classified due to a motion blur.

The classification section 310 may determine whether or not each pixel or each area agrees with the characteristics of a normal structure to classify each pixel or each area as the normal part or the non-normal part (abnormal part). The enhancement processing section 330 may exclude the pixel or the area for which it has been determined that the motion amount is larger than the threshold value from the target of the enhancement process based on the classification result that represents the normal part or the non-normal part.

The object in which a normal pit pattern is present may be erroneously classified as the non-normal part in an area in which the image is blurred due to a motion blur. According to the second embodiment, since the (normal/non-normal) enhancement process is not performed in an area in which the motion amount is large, it is possible to present highly reliable (normal/non-normal) classification results to the user even when the object has been erroneously classified.

4. Modification of second embodiment

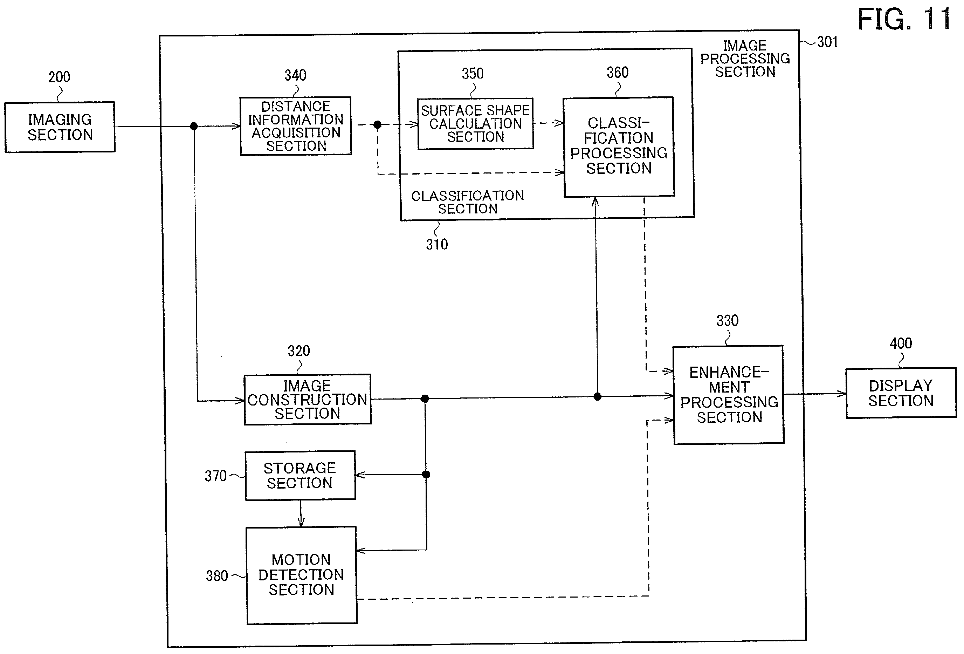

FIG. 11 illustrates a configuration example of an image processing section 301 according to a modification of the second embodiment. The image processing section 301 includes a classification section 310, an image construction section 320, an enhancement processing section 330, a distance information acquisition section 340, a storage section 370, and a motion detection section 380.

In the modification, the motion determination section 390 is omitted, and the motion detection section 380 is connected to the enhancement processing section 330. The enhancement processing section 330 controls the enhancement level based on the motion information. Specifically, the enhancement processing section 330 decreases the enhancement level applied to a pixel as the motion amount increases. For example, when enhancing the abnormal part, the degree of enhancement of the abnormal part decreases as the motion amount increases.

According to the modification, the enhancement processing section 330 decreases the enhancement level of the enhancement process applied to each pixel or each area within the captured image as the motion amount of the object increases based on the motion information.

Since the image normally becomes less clear as the motion amount of the object increases, it is considered that the reliability of the matching process decreases as the motion amount of the object increases. According to the modification, since the enhancement level can be decreased as the reliability of the classification results decreases, it is possible to prevent a situation in which unreliable classification results are also presented to the user.

5. Third embodiment

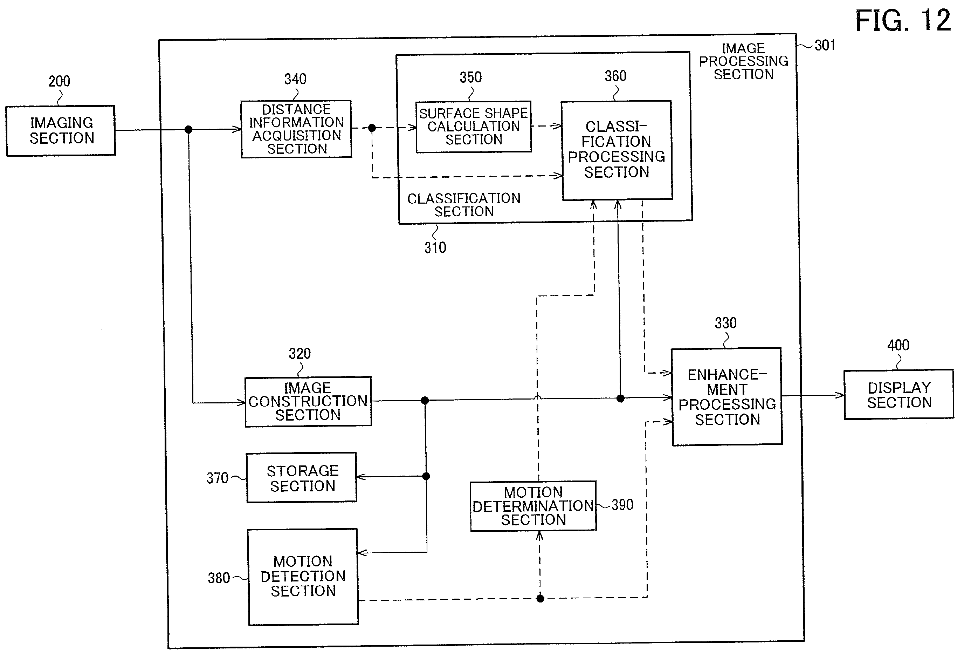

FIG. 12 illustrates a configuration example of an image processing section 301 according to a third embodiment. The image processing section 301 includes a classification section 310, an image construction section 320, an enhancement processing section 330, a distance information acquisition section 340, a storage section 370, a motion detection section 380, and a motion determination section 390.

In the third embodiment, the motion detection section 380 is connected to the motion determination section 390 and the enhancement processing section 330, and the motion determination section 390 is connected to the classification section 310. The classification section 310 does not classify an area in which the motion amount is large as "normal part" or "abnormal part" (i.e., classifies an area in which the motion amount is large as "unknown") in the same manner as in the first embodiment. The enhancement processing section 330 decreases the enhancement level applied to an area in which the motion amount is large in the same manner as in the modification of the second embodiment.

6. Fourth embodiment

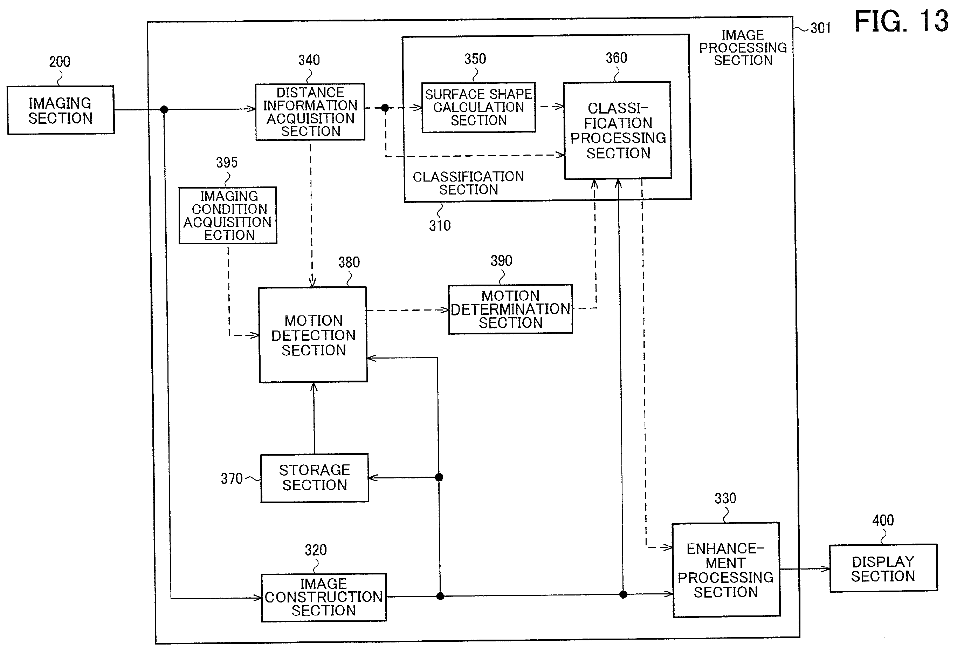

FIG. 13 illustrates a configuration example of an image processing section 301 according to a fourth embodiment. The image processing section 301 includes a classification section 310, an image construction section 320, an enhancement processing section 330, a distance information acquisition section 340, a storage section 370, a motion detection section 380, a motion determination section 390, and an imaging condition acquisition section 395.

The first embodiment illustrates an example in which the motion amount within the image is detected as the motion information. In the fourth embodiment, the motion amount based on the object is detected. Note that the motion detection process according to the fourth embodiment can also be applied to the first to third embodiments.

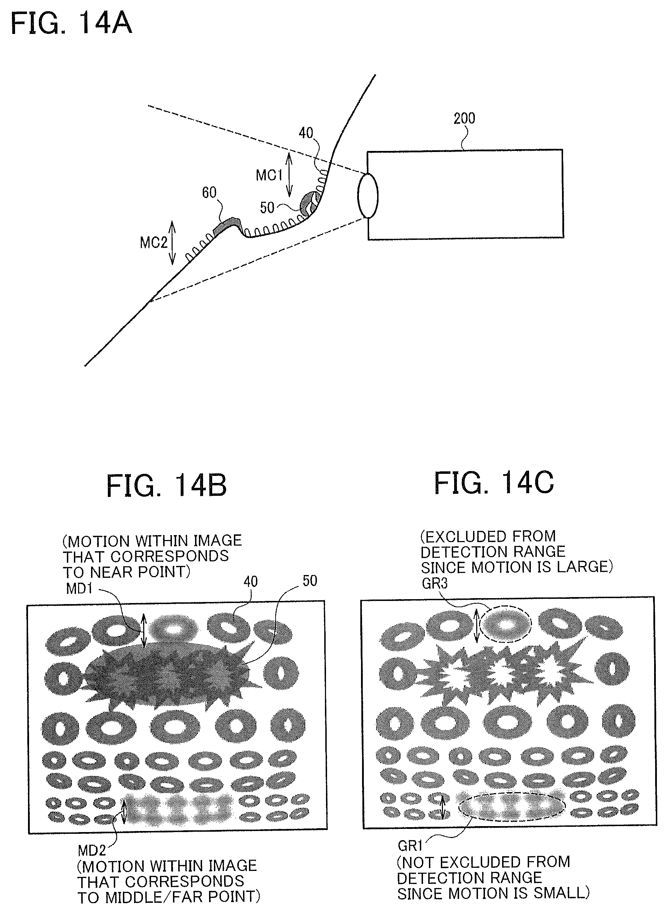

The operation according to the fourth embodiment is described below with reference to FIGS. 14A to 14C. FIG. 14A illustrates the relationship between the imaging section 200 and the object. FIGS. 14B and 14C illustrate an example of the acquired image.

As illustrated in FIG. 14A, the operator (user) brings the imaging section 200 closer to the object. In this case, the operator moves the imaging section 200 so that the imaging section 200 directly faces the object (abnormal part (abnormal duct 50 and duct disappearance area 60)). However, it may be impossible to cause the imaging section 200 to directly face the object in a narrow area inside the body. In such a case, an image is captured diagonally with respect to the object. In this case, the object situated at the near point is displayed to have a large size (see the upper part of the image), and the object situated at the middle/far point is displayed to have a small size (see the lower part of the image) (see FIG. 14B). If the object situated at the near point has made a motion MC1, and the object situated at the middle/far point has made a motion MC2 that is almost equal to the motion MC1 (see FIG. 14A), a motion amount MD2 within the image that corresponds to the middle/far point is detected to be smaller than a motion amount MD1 within the image that corresponds to the near point (see FIG. 14B).

In the first embodiment, since the classification section 310 sets the target range of the classification process based on the motion amount within the image, the object situated at the middle/far point is included in the target range of the classification process when the above situation has occurred. Specifically, an area GR3 that corresponds to the near point is classified as "unknown", and the (normal/abnormal) classification process is performed on an area GR1 that corresponds to the middle/far point since the motion amount MD2 within the image is small (see FIG. 14C). However, since the structure of the object situated at the middle/far point is displayed to have a small size, the structure is unclearly displayed although the motion amount MD2 is small. Therefore, the object situated at the middle/far point may be erroneously detected to be an abnormal part when the detection range is set based on the motion amount within the image.

In the fourth embodiment, the motion detection section 380 detects the motion amount based on the object, and the classification section 310 sets the target range of the classification process based on the motion amount. This makes it possible to suppress a situation in which the object situated at the middle/far point is erroneously classified due to a motion.

More specifically, the image processing section 301 according to the fourth embodiment further includes the imaging condition acquisition section 395. The imaging condition acquisition section 395 is connected to the motion detection section 380. The distance information acquisition section 340 is connected to the motion detection section 380. The control section 302 (not illustrated in FIG. 13) is bidirectionally connected to each section of the image processing section 301, and controls each section of the image processing section 301.

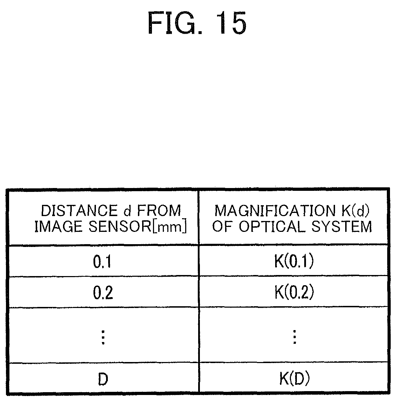

The imaging condition acquisition section 395 acquires the imaging condition (employed when the image was captured) from the control section 302. Specifically, the imaging condition acquisition section 395 acquires the magnification $K(d)$ of the optical system of the imaging section 200. For example, when the optical system is a fixed-focus optical system, the magnification $K(d)$ of the optical system corresponds to the distance d from the image sensor to the object on a one-to-one basis (see FIG. 15). The magnification $K(d)$ corresponds to the image magnification. The magnification $K(d)$ decreases (i.e., the size of the object within the image decreases) as the distance d increases.

Note that the optical system is not limited to a fixed-focus optical system. The optical system may be a variable-focus optical system (that can implement optical zoom). In this case, the table illustrated in FIG. 15 is provided corresponding to each zoom lens position (zoom magnification) of the optical system, and the magnification $K(d)$ is acquired by referring to the table that corresponds to the zoom lens position of the optical system when the image was captured.

The motion detection section 380 detects the motion amount within the image, and detects the motion amount of the object based on the detected motion amount within the image, the distance information, and the imaging condition. Specifically, the motion detection section 380 detects the motion amount $Mv(x, y)$ within the image at the coordinates $(x, y)$ of each pixel (see the first embodiment). The motion detection section 380 acquires the distance information $d(x, y)$ about the distance to the object at each pixel from the distance map, and acquires the magnification $K(d(x, y))$ of the optical system that corresponds to the distance information d(x, y) from the table. The motion detection section 380 multiplies the motion amount Mv(x, y) by the magnification K(d(x, y)) to calculate the motion amount Mvobj(x, y) based on the object at the coordinates (x, y) (see the following expression (4)). The motion detection section 380 transmits the calculated motion amount Mvobj(x, y) of the object to the classification section 310 as the motion information.

$$\mathrm{Mvobj(x,\ y)=Mv(x,\ y)\times K(d(x,\ y))} \qquad (4)$$

According to the fourth embodiment, since the target range of the (normal/abnormal) classification process is set based on the motion amount Mvobj(x, y) based on the object, it is possible to suppress a situation in which an abnormal part is erroneously detected due to a motion blur, irrespective of the distance (distance information d(x, y)) to the object.

7. First classification method

7.1. Classification section

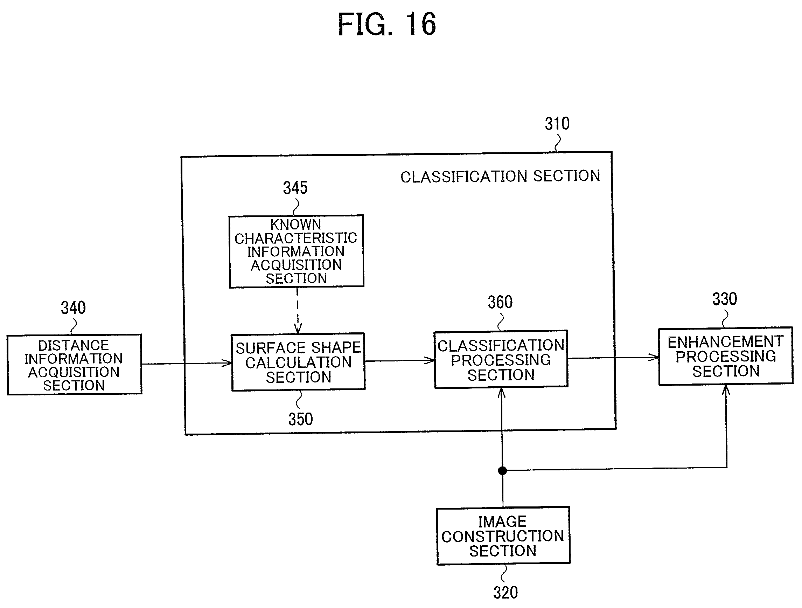

The classification process performed by the classification section 310 according to the first to fourth embodiments is described in detail below. FIG. 16 illustrates a detailed configuration example of the classification section 310. The classification section 310 includes a known characteristic information acquisition section 345, the surface shape calculation section 350, and the classification processing section 360.

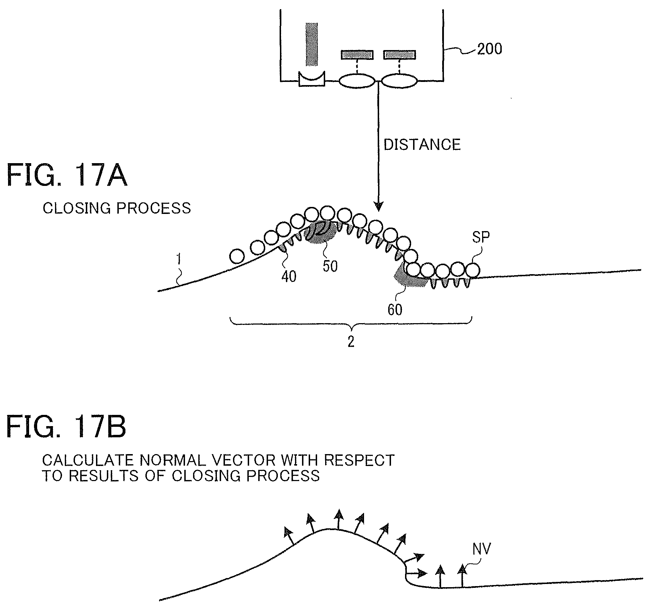

The operation of the classification section 310 is described below taking an example in which the observation target is the large intestine. As illustrated in FIG. 17A, a polyp 2 (i.e., elevated lesion) is present on the surface 1 of the large intestine (i.e., observation target), and a normal duct 40 and an abnormal duct 50 are present in the surface layer of the mucous membrane of the polyp 2. A recessed lesion 60 (in which the ductal structure has disappeared) is present at the base of the polyp 2. As illustrated in FIG. 1B, when the polyp 2 is viewed from above, the normal duct 40 has an approximately circular shape, and the abnormal duct 50 has a shape differing from that of the normal duct 40.

The surface shape calculation section 350 performs a closing process or an adaptive low-pass filtering process on the distance information (e.g., distance map) input from the distance information acquisition section 340 to extract a structure having a size equal to or larger than that of a given structural element. The given structural element is the classification target ductal structure (pit pattern) formed on the surface 1 of the observation target part.

Specifically, the known characteristic information acquisition section 345 acquires structural element information as the known characteristic information, and outputs the structural element information to the surface shape calculation section 350. The structural element information is size information that is determined by the optical magnification of the imaging section 200, and the size (width information) of the ductal structure to be classified from the surface structure of the surface 1. Specifically, the optical magnification is determined corresponding to the distance to the object, and the size of the ductal structure within the image captured at a specific distance to the object is acquired as the structural element information by performing a size adjustment process using the optical magnification.

For example, the control section 302 included in the processor section 300 stores a standard size of a ductal structure, and the known characteristic information acquisition section 345 acquires the standard size from the control section 302, and performs the size adjustment process using the optical magnification. Specifically, the control section 302 determines the observation target part based on the scope ID information input from the memory 210 included in the imaging section 200. For example, when the imaging section 200 is an upper gastrointestinal scope, the observation target part is determined to be the gullet, the stomach, or the duodenum. When the imaging section 200 is a lower gastrointestinal scope, the observation target part is determined to be the large intestine. A standard duct size corresponding to each observation target part is stored in the control section 302 in advance. When the external I/F section 500 includes a switch that can be operated by the user for selecting the observation target part, the user may select the observation target part by operating the switch, for example.

The surface shape calculation section 350 adaptively generates surface shape calculation information based on the input distance information, and calculates the surface shape information about the object using the surface shape calculation information. The surface shape information represents the normal vector NV illustrated in FIG. 17B, for example. The details of the surface shape calculation information are described later. For example, the surface shape calculation information may be the morphological kernel size (i.e., the size of the structural element) that is adapted to the distance information at the attention position (position in question) on the distance map, or may be the low-pass characteristics of a filter that is adapted to the distance information. Specifically, the surface shape calculation information is information that adaptively changes the characteristics of a nonlinear or linear low-pass filter corresponding to the distance information.

The surface shape information thus generated is input to the classification processing section 360 together with the distance map. As illustrated in FIGS. 18A and 18B, the classification processing section 360 generates a corrected pit (classification reference) from a basic pit corresponding to the three-dimensional shape of the surface of tissue captured within the captured image. The basic pit is generated by modeling a normal ductal structure for classifying a ductal structure. The basic pit is a binary image, for example. The terms "basic pit" and "corrected pit" are used since the pit pattern is the classification target. Note that the terms "basic pit" and "corrected pit" can respectively be replaced by the terms "reference pattern" and "corrected pattern" having a broader meaning.

The classification processing section 360 performs the classification process using the generated classification reference (corrected pit). Specifically, the image output from the image construction section 320 is input to the classification processing section 360. The classification processing section 360 determines the presence or absence of the corrected pit within the captured image using a known pattern matching process, and outputs a classification map (in which the classification areas are grouped) to the enhancement processing section 330. The classification map is a map in which the captured image is classified into an area that includes the corrected pit and an area other than the area that includes the corrected pit. For example, the classification map is a binary image in which "1" is assigned to pixels included in an area that includes the corrected pit, and "0" is assigned to pixels included in an area other than the area that includes the corrected pit. When the object is classified as "unknown" corresponding to the motion amount, "2" may be assigned to pixels included in an area that is classified as "unknown" (i.e., a ternary image may be used).

The image (having the same size as that of the classification image) output from the image construction section 320 is input to the enhancement processing section 330. The enhancement processing section 330 performs the enhancement process on the image output from the image construction section 320 using the information that represents the classification results.

7.2. Surface shape calculation section

The process performed by the surface shape calculation section 350 is described below with reference to FIGS. 17A and 17B.

FIG. 17A is a cross-sectional view illustrating the surface 1 of the object and the imaging section 200 taken along the optical axis of the imaging section 200. FIG. 17A schematically illustrates a state in which the surface shape is calculated using the morphological process (closing process). The radius of a sphere SP (structural element) used for the closing process is set to be equal to or more than twice the size of the classification target ductal structure (surface shape calculation information), for example. The size of the ductal structure has been adjusted to the size within the image corresponding to the distance to the object corresponding to each pixel (see above).

It is possible to extract the three-dimensional surface shape of the smooth surface 1 without extracting the minute concavities and convexities of the normal duct 40, the abnormal duct 50, and the duct disappearance area 60 by utilizing the sphere SP having such a size. This makes it possible to reduce a correction error as compared with the case of correcting the basic pit using the surface shape in which the minute concavities and convexities remain.

FIG. 18B is a cross-sectional view illustrating the surface of tissue after the closing process has been performed. FIG. 18B illustrates the results of a normal vector (NV) calculation process performed on the surface of tissue. The normal vector NV is used as the surface shape information. Note that the surface shape information is not limited to the normal vector NV. The surface shape information may be the curved surface illustrated in FIG. 18B, or may be another piece of information that represents the surface shape.

The known characteristic information acquisition section 345 acquires the size (e.g., the width in the longitudinal direction) of the duct of tissue as the known characteristic information, and determines the radius (corresponding to the size of the duct within the image) of the sphere SP used for the closing process. In this case, the radius of the sphere SP is set to be larger than the size of the duct within the image. The surface shape calculation section 350 can extract the desired surface shape by performing the closing process using the sphere SP.

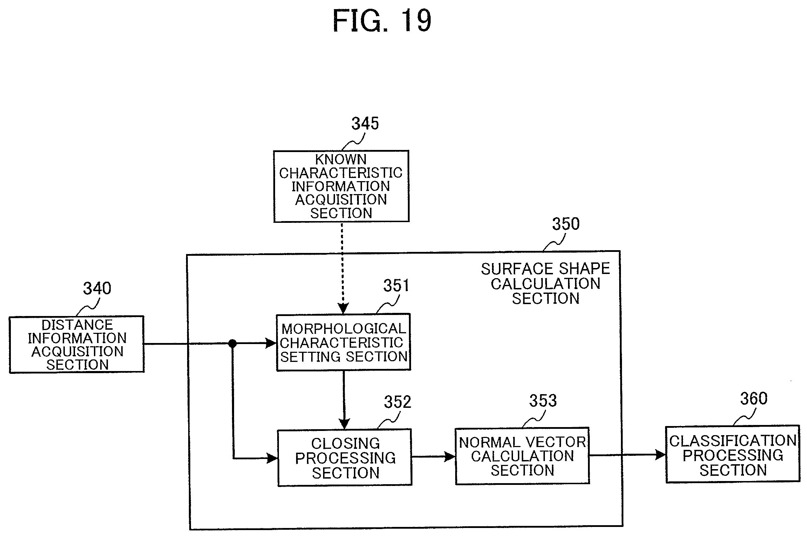

FIG. 19 illustrates a detailed configuration example of the surface shape calculation section 350. The surface shape calculation section 350 includes a morphological characteristic setting section 351, a closing processing section 352, and a normal vector calculation section 353.

The size (e.g., the width in the longitudinal direction) of the duct of tissue (i.e., known characteristic information) is input to the morphological characteristic setting section 351 from the known characteristic information acquisition section 345. The morphological characteristic setting section 351 determines the surface shape calculation information (e.g., the radius of the sphere SP used for the closing process) based on the size of the duct and the distance map.

The information about the radius of the sphere SP thus determined is input to the closing processing section 352 as a radius map having the same number of pixels as that of the distance map, for example. The radius map is a map in which the information about the radius of the sphere SP corresponding to each pixel is linked to each pixel. The closing processing section 352 performs the closing process while changing the radius of the sphere SP on a pixel basis using the radius map, and outputs the processing results to the normal vector calculation section 353.

The distance map obtained by the closing process is input to the normal vector calculation section 353. The normal vector calculation section 353 defines a plane using three-dimensional information (e.g., the coordinates of the pixel and the distance information at the corresponding coordinates) about the attention sampling position (sampling position in question) and two sampling positions adjacent thereto on the distance map, and calculates the normal vector to the defined plane. The normal vector calculation section 353 outputs the calculated normal vector to the classification processing section 360 as a normal vector map that is identical with the distance map as to the number of sampling points.

7.3. Classification processing section

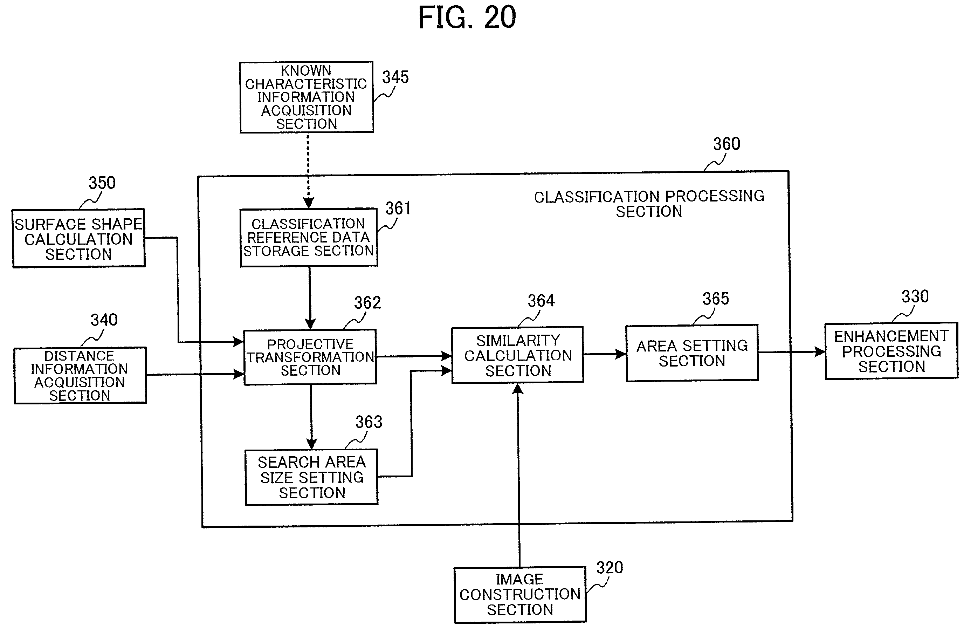

FIG. 20 illustrates a detailed configuration example of the classification processing section 360. The classification processing section 360 includes a classification reference data storage section 361, a projective transformation section 362, a search area size setting section 363, a similarity calculation section 364, and an area setting section 365.

The classification reference data storage section 361 stores the basic pit obtained by modeling the normal duct exposed on the surface of tissue (see FIG. 18A). The basic pit is a binary image having a size corresponding to the size of the normal duct captured at a given distance. The classification reference data storage section 361 outputs the basic pit to the projective transformation section 362.

The distance map output from the distance information acquisition section 340, the normal vector map output from the surface shape calculation section 350, and the optical magnification output from the control section 302 (not illustrated in FIG. 20) are input to the projective transformation section 362. The projective transformation section 362 extracts the distance information that corresponds to the attention sampling position from the distance map, and extracts the normal vector at the sampling position corresponding thereto from the normal vector map. The projective transformation section 362 subjects the basic pit to projective transformation using the normal vector, and performs a magnification correction process corresponding to the optical magnification to generate a corrected pit (see FIG. 18B). The projective transformation section 362 outputs the corrected pit to the similarity calculation section 364 as the classification reference, and outputs the size of the corrected pit to the search area size setting section 363.

The search area size setting section 363 sets an area having a size twice the size of the corrected pit to be a search area used for a similarity calculation process, and outputs the information about the search area to the similarity calculation section 364.

The similarity calculation section 364 receives the corrected pit at the attention sampling position from the projective transformation section 362, and receives the search area that corresponds to the corrected pit from the search area size setting section 363. The similarity calculation section 364 extracts the image of the search area from the image input from the image construction section 320.

The similarity calculation section 364 performs a high-pass filtering process or a band-pass filtering process on the extracted image of the search area to remove a low-frequency component, and performs a binarization process on the resulting image to generate a binary image of the search area. The similarity calculation section 364 performs a pattern matching process on the binary image of the search area using the corrected pit to calculate a correlation value, and outputs the peak position of the correlation value and a maximum correlation value map to the area setting section 365. The correlation value is the sum of absolute differences, and the maximum correlation value is the minimum value of the sum of absolute differences, for example.

Note that the correlation value may be calculated using a phase-only correlation (POC) method or the like. Since rotation and a change in magnification are invariable when using the POC method, it is possible to improve the correlation calculation accuracy.

The area setting section 365 calculates an area for which the sum of absolute differences is equal to or less than a given threshold value T based on the maximum correlation value map input from the similarity calculation section 364, and calculates the three-dimensional distance between the position within the calculated area that corresponds to the maximum correlation value and the position within the adjacent search range that corresponds to the maximum correlation value. When the calculated three-dimensional distance is included within a given error range, the area setting section 365 groups an area that includes the maximum correlation position as a normal area to generate a classification map. The area setting section 365 outputs the generated classification map to the enhancement processing section 330.

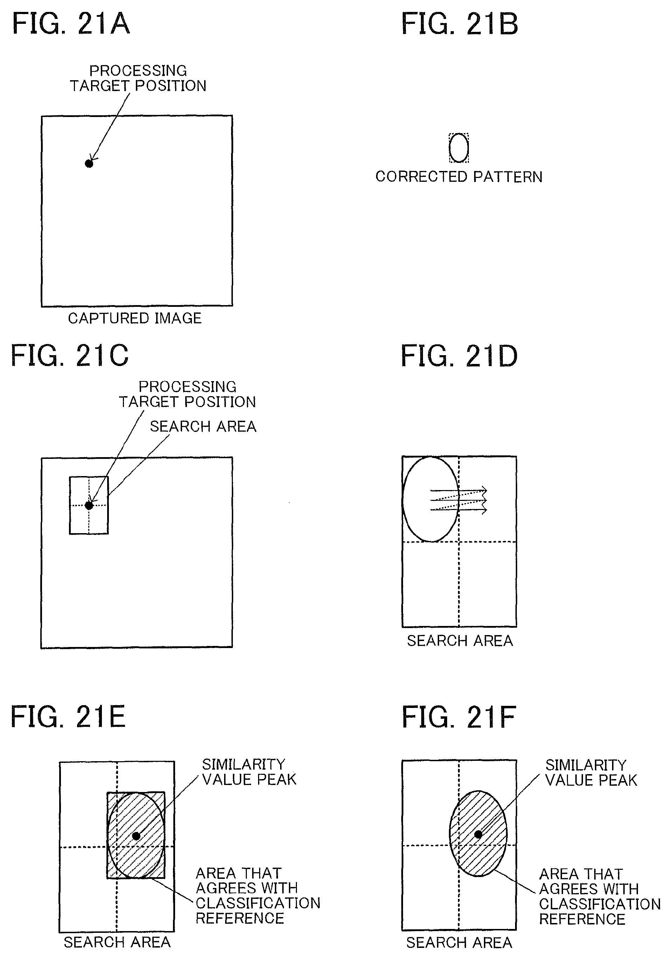

FIGS. 21A to 21F illustrate a specific example of the classification process. As illustrated in FIG. 21A, one position within the image is set to be the processing target position. The projective transformation section 362 acquires a corrected pattern at the processing target position by deforming the reference pattern based on the surface shape information that corresponds to the processing target position (see FIG. 21B). The search area size setting section 363 sets the search area (e.g., an area having a size twice the size of the corrected pit pattern) around the processing target position using the acquired corrected pattern (see FIG. 21C).

The similarity calculation section 364 performs the matching process on the captured structure and the corrected pattern within the search area (see FIG. 21D). When the matching process is performed on a pixel basis, the similarity is calculated on a pixel basis. The area setting section 365 determines a pixel that corresponds to the similarity peak within the search area (see FIG. 21E), and determines whether or not the similarity at the determined pixel is equal to or larger than a given threshold value. When the similarity at the determined pixel is equal to or larger than the threshold value (i.e., when the corrected pattern has been detected within the area having the size of the corrected pattern based on the peak position (the center of the corrected pattern is set to be the reference position in FIG. 21E)), it is determined that the area agrees with the reference pattern.

Note that the inside of the shape that represents the corrected pattern may be determined to be the area that agrees with the classification reference (see FIG. 21F). Various other modifications may also be made. When the similarity at the determined pixel is less than the threshold value, it is determined that a structure that agrees with the reference pattern is not present in the area around the processing target position. An area (0, 1, or a plurality of areas) that agrees with the reference pattern, and an area other than the area that agrees with the reference pattern are set within the captured image by performing the above process corresponding to each position within the image. When a plurality of areas agree with the reference pattern, overlapping areas and contiguous areas among the plurality of areas are integrated to obtain the final classification results. Note that the classification process based on the similarity described above is only an example. The classification process may be performed using another method. The similarity may be calculated using various known methods that calculate the similarity between images or the difference between images, and detailed description thereof is omitted.

According to the fourth embodiment, the classification section 310 includes the surface shape calculation section 350 that calculates the surface shape information about the object based on the distance information and the known characteristic information, and the classification processing section 360 that generates the classification reference based on the surface shape information, and performs the classification process that utilizes the generated classification reference.

This makes it possible to adaptively generate the classification reference based on the surface shape represented by surface shape information, and perform the classification process. A decrease in the accuracy of the classification process due to the surface shape may occur due to deformation of the structure within the captured image caused by the angle formed by the optical axis (optical axis direction) of the imaging section 200 and the surface of the object, for example. The method according to the fourth embodiment makes it possible to accurately perform the classification process even in such a situation.

The known characteristic information acquisition section 345 may acquire the reference pattern that corresponds to the structure of the object in a given state as the known characteristic information, and the classification processing section 360 may generate the corrected pattern as the classification reference, and perform the classification process using the generated classification reference, the corrected pattern being acquired by performing a deformation process based on the surface shape information on the reference pattern.

This makes it possible to accurately perform the classification process even when the structure of the object has been captured in a deformed state corresponding to the surface shape. Specifically, a circular ductal structure may be captured in a variously deformed state (see FIG. 1B, for example). It is possible to appropriately detect and classify the pit pattern even in a deformed area by generating an appropriate corrected pattern (corrected pit in FIG. 18B) from the reference pattern (basic pit in FIG. 18A) corresponding to the surface shape, and utilizing the generated corrected pattern as the classification reference.

The known characteristic information acquisition section 345 may acquire the reference pattern that corresponds to the structure of the object in a normal state as the known characteristic information.

This makes it possible to implement the classification process that classifies the captured image into a normal area and an abnormal area. The term "abnormal area" refers to an area that is suspected to be a lesion when using a medical endoscope, for example. Since it is considered that the user normally pays attention to such an area, a situation in which an area to which attention should be paid is missed can be suppressed by appropriately classifying the captured image, for example.

The object may include a global three-dimensional structure, and a local concave-convex structure that is more local than the global three-dimensional structure, and the surface shape calculation section 350 may calculate the surface shape information by extracting the global three-dimensional structure among the global three-dimensional structure and the local concave-convex structure included in the object from the distance information.

This makes it possible to calculate the surface shape information from the global structure when the structures of the object are classified into a global structure and a local structure. Deformation of the reference pattern within the captured image predominantly occurs due to a global structure that is larger than the reference pattern. Therefore, it is possible to accurately perform the classification process by calculating the surface shape information from the global three-dimensional structure.

8. Second classification method

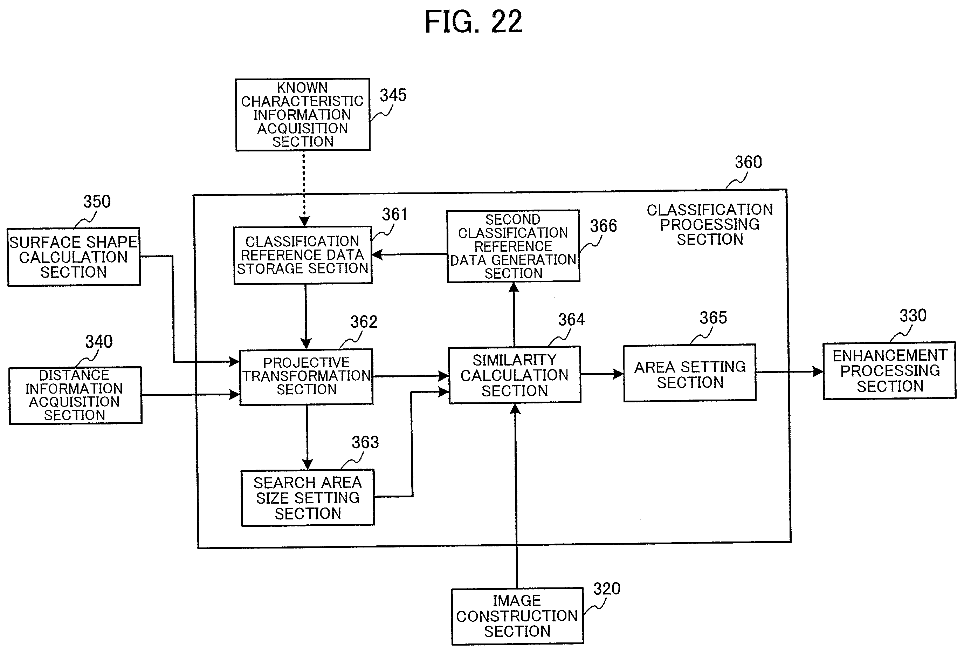

FIG. 22 illustrates a detailed configuration example of a classification processing section 360 that implements a second classification method. The classification processing section 360 includes a classification reference data storage section 361, a projective transformation section 362, a search area size setting section 363, a similarity calculation section 364, an area setting section 365, and a second classification reference data generation section 366. Note that the same elements as those described above in connection with the first classification method are indicated by the same reference signs (symbols), and description thereof is appropriately omitted.

The second classification method differs from the first classification method in that the basic pit (classification reference) is provided corresponding to the normal duct and the abnormal duct, a pit is extracted from the actual captured image, and used as second classification reference data (second reference pattern), and the similarity is calculated based on the second classification reference data.

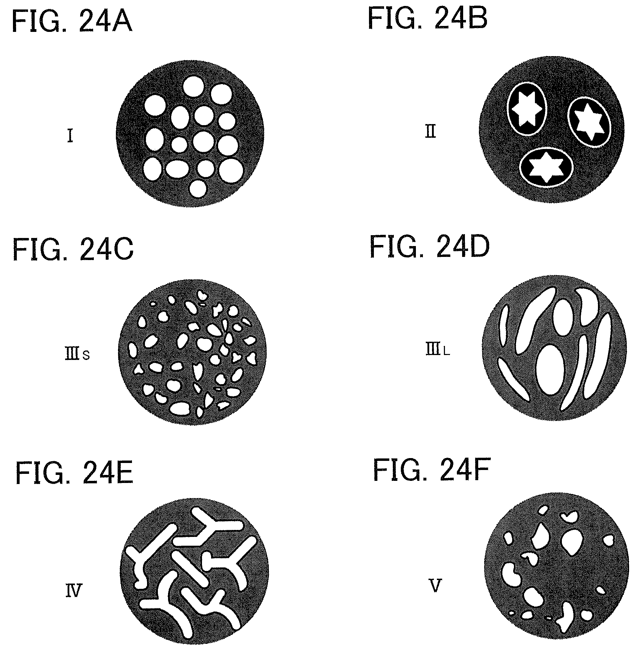

As illustrated in FIGS. 24A to 24F, the shape of a pit pattern on the surface of tissue changes corresponding to the state (normal state or abnormal state) of the pit pattern, the stage of lesion progression (when the state of the pit pattern is an abnormal state), and the like. For example, the pit pattern of a normal mucous membrane has an approximately circular shape (see FIG. 24A). The pit pattern has a complex shape (e.g., star-like shape (see FIG. 24B) or tubular shape (see FIGS. 24C and 24D)) when the lesion has advanced, and may disappear (see FIG. 24F) when the lesion has further advanced. Therefore, it is possible to determine the state of the object by storing these typical patterns as a reference pattern, and determining the similarity between the surface of the object captured within the captured image and the reference pattern, for example.

The differences from the first classification method are described in detail below. A plurality of pits including the basic pit corresponding to the normal duct (see FIG. 23) are stored in the classification reference data storage section 361, and output to the projective transformation section 362. The process performed by the projective transformation section 362 is the same as described above in connection with the first classification method. Specifically, the projective transformation section 362 performs the projective transformation process on each pit stored in the classification reference data storage section 361, and outputs the corrected pits corresponding to a plurality of classification types to the search area size setting section 363 and the similarity calculation section 364.

The similarity calculation section 364 generates the maximum correlation value map corresponding to each corrected pit. Note that the maximum correlation value map is not used to generate the classification map (i.e., the final output of the classification process), but is output to the second classification reference data generation section 366, and used to generate additional classification reference data.

The second classification reference data generation section 366 sets the pit image at a position within the image for which the similarity calculation section 364 has determined that the similarity is high (i.e., the absolute difference is equal to or smaller than a given threshold value) to be the classification reference. This makes it possible to implement a more optimum and accurate classification (determination) process since the pit extracted from the actual image is used as the classification reference instead of using a typical pit model provided in advance.

More specifically, the maximum correlation value map (corresponding to each type) output from the similarity calculation section 364, the image output from the image construction section 320, the distance map output from the distance information acquisition section 340, the optical magnification output from the control section 302, and the duct size (corresponding to each type) output from the known characteristic information acquisition section 345 are input to the second classification reference data generation section 366. The second classification reference data generation section 366 extracts the image data corresponding to the maximum correlation value sampling position (corresponding to each type) based on the distance information that corresponds to the maximum correlation value sampling position, the size of the duct, and the optical magnification.

The second classification reference data generation section 366 acquires a grayscale image (that cancels the difference in brightness) obtained by removing a low-frequency component from the extracted (actual) image, and outputs the grayscale image to the classification reference data storage section 361 as the second classification reference data together with the normal vector and the distance information. The classification reference data storage section 361 stores the second classification reference data and the relevant information. The second classification reference data having a high correlation with the object has thus been collected corresponding to each type.

Note that the second classification reference data includes the effects of the angle formed by the optical axis (optical axis direction) of the imaging section 200 and the surface of the object, and the effects of deformation (change in size) corresponding to the distance from the imaging section 200 to the surface of the object. The second classification reference data generation section 366 may generate the second classification reference data after performing a process that cancels these effects. Specifically, the results of the deformation process (projective transformation process and scaling process) performed on the grayscale image so as to achieve a state in which the image is captured at a given distance in a given reference direction may be used as the second classification reference data.

After the second classification reference data has been generated, the projective transformation section 362, the search area size setting section 363, and the similarity calculation section 364 perform the process on the second classification reference data. Specifically, the projective transformation process is performed on the second classification reference data to generate a second corrected pattern, and the process described above in connection with the first classification method is performed using the generated second corrected pattern as the classification reference.

Note that the basic pit corresponding to the abnormal duct used in connection with the second classification method is not normally point-symmetrical. Therefore, it is desirable that the similarity calculation section 364 calculate the similarity (when using the corrected pattern or the second corrected pattern) by performing a rotation-invariant phase-only correction (POC) process.

The area setting section 365 generates the classification map in which the pits are grouped on a class basis (type I, type II, ...) (see FIG. 23), or generates the classification map in which the pits are grouped on a type basis (type A, type B, ...) (see FIG. 23). Specifically, the area setting section 365 generates the classification map of an area in which a correlation is obtained by the corrected pit classified as the normal duct, and generates the classification map of an area in which a correlation is obtained by the corrected pit classified as the abnormal duct on a class basis or a type basis. The area setting section 365 synthesizes these classification maps to generate a synthesized classification map (multi-valued image). In this case, the overlapping area of the areas in which a correlation is obtained corresponding to each class may be set to be an unclassified area, or may be set to the type having a higher malignant level. The area setting section 365 outputs the synthesized classification map to the enhancement processing section 330.

The enhancement processing section 330 performs the luminance or color enhancement process based on the classification map (multi-valued image), for example.

According to the fourth embodiment, the known characteristic information acquisition section 345 acquires the reference pattern that corresponds to the structure of the object in an abnormal state as the known characteristic information.

This makes it possible to acquire a plurality of reference patterns (see FIG. 23), generate the classification reference using the plurality of reference patterns, and perform the classification process, for example. Specifically, the state of the object can be finely classified by performing the classification process using the typical patterns illustrated in FIGS. 24A to 24F as the reference pattern.

The known characteristic information acquisition section 345 may acquire the reference pattern that corresponds to the structure of the object in a given state as the known characteristic information, and the classification processing section 360 may perform the deformation process based on the surface shape information on the reference pattern to acquire the corrected pattern, calculate the similarity between the structure of the object captured within the captured image and the corrected pattern corresponding to each position within the captured image, and acquire a second reference pattern candidate based on the calculated similarity. The classification processing section 360 may generate the second reference pattern as a new reference pattern based on the acquired second reference pattern candidate and the surface shape information, perform the deformation process based on the surface shape information on the second reference pattern to generate the second corrected pattern as the classification reference, and perform the classification process using the generated classification reference.

This makes it possible to generate the second reference pattern based on the captured image, and perform the classification process using the second reference pattern. Since the classification reference can be generated from the object that is captured within the captured image, the classification reference sufficiently reflects the characteristics of the object (processing target), and it is possible to improve the accuracy of the classification process as compared with the case of directly using the reference pattern acquired as the known characteristic information.

Although only some embodiments of the invention and the modifications thereof have been described in detail above, those skilled in the art will readily appreciate that many modifications are possible in the embodiments and the modifications thereof without materially departing from the novel teachings and advantages of the invention. A plurality of elements described in connection with the above embodiments and the modifications thereof may be appropriately combined to implement various configurations. For example, some elements may be omitted from the elements described in connection with the above embodiments and the modifications thereof. Some of the elements described in connection with different embodiments or modifications thereof may be appropriately combined. Specifically, various modifications and applications are possible without materially departing from the novel teachings and advantages of the invention. Any term cited with a different term having a broader meaning or the same meaning at least once in the specification and the drawings can be replaced by the different term in any place in the specification and the drawings.

REFERENCE SIGNS LIST

40: normal duct, 50: abnormal duct, 60: duct disappearance area, 100: light source section, 101: white light source, 102: rotary color filter, 103: rotation driver section, 104: condenser lens, 200: imaging section, 201: light guide fiber, 203: illumination lens, 204: objective lens, 206, 207: image sensor, 209: A/D conversion section, 210: memory, 212: connector, 300: processor section, 301: image processing section, 302: control section, 305: image acquisition section, 310: classification section, 320: image construction section, 330: enhancement processing section, 340: distance information acquisition section, 345: known characteristic information acquisition section, 350: surface shape calculation section, 351: morphological characteristic setting section, 352: closing processing section, 353: normal vector calculation section, 360: classification processing section, 361: classification reference data storage section, 362: projective transformation section, 363: search area size setting section, 364: similarity calculation section, 365: area setting section, 366: classification reference data generation section, 370: storage section, 380: motion detection section, 390: motion determination section, 395: imaging condition acquisition section, 400: display section, 500: external I/F section, GR1 to GR3: area, MA, MC1, MC2: motion, MB: motion blur, MD1, MD2: motion amount, NV: normal vector, SP: sphere

**Claims**

1. An image processing device comprising:

   an image acquisition section that acquires a captured image in time series, the captured image including an image of an object;
   a distance information acquisition section that acquires distance information based on a distance from an imaging section to the object when the imaging section captured the captured image;
   a motion detection section that detects motion information about a local motion of the object based on the captured image acquired in time series;
   a classification section that performs a classification process that classifies a structure of the object based on the distance information; and
   an enhancement processing section that performs an enhancement process on the captured image based on results of the classification process, and controls a target or an enhancement level of the enhancement process corresponding to the motion information about the local motion of the object.

2. The image processing device as defined in claim 1, further comprising:

   a motion determination section that determines whether or not a motion amount of the object within a pixel or an area within the captured image is larger than a threshold value based on the motion information,
   the enhancement processing section excluding the pixel or the area for which it has been determined that the motion amount is larger than the threshold value from the target of the enhancement process based on a classification result.

3. The image processing device as defined in claim 2,
   the classification section determining whether or not the pixel or the area agrees with characteristics of a normal structure to classify the pixel or the area as a normal part or a non-normal part, and
   the enhancement processing section excluding the pixel or the area for which it has been determined that the motion amount is larger than the threshold value from the target of the enhancement process based on a classification result that represents the normal part or the non-normal part.

4. The image processing device as defined in claim 1,
   the enhancement processing section including an enhancement control section that decreases the enhancement level of the enhancement process applied to the pixel or the area within the captured image as the motion amount of the object within the pixel or the area increases based on the motion information.

5. The image processing device as defined in claim 4, further comprising:

   a motion determination section that determines whether or not the motion amount of the object within the pixel or the area within the captured image is larger than a threshold value based on the motion information,
   the classification section excluding the pixel or the area from a target of the classification process when the motion determination section has determined that the motion amount is larger than the threshold value.

6. The image processing device as defined in claim 1,
   the motion detection section converting the motion information detected based on the captured image into the motion information based on the object based on the distance information, and
   the enhancement processing section controlling the target or the enhancement level of the enhancement process corresponding to the motion information based on the object.

7. The image processing device as defined in claim 6,
   the motion detection section including an imaging condition acquisition section that acquires an imaging condition employed when the captured image was captured, and
the motion detection section calculating the motion information based on the object based on the distance information and the imaging condition.

8. The image processing device as defined in claim 7,
the imaging condition being a magnification of an optical system of the imaging section that corresponds to the distance information, and
the motion detection section calculating the motion information based on the object by multiplying the motion information detected based on the captured image by the magnification.

9. An image processing device comprising:

an image acquisition section that acquires a captured image in time series, the captured image including an image of an object;
a distance information acquisition section that acquires distance information based on a distance from an imaging section to the object when the imaging section captured the captured image;
a motion detection section that detects motion information about a local motion of the object based on the captured image acquired in time series; and
a classification section that performs a classification process that classifies a structure of the object based on the distance information, and controls a target of the classification process corresponding to the motion information about the local motion of the object.

10. The image processing device as defined in claim 9, further comprising:

a motion determination section that determines whether or not a motion amount of the object within a pixel or an area within the captured image is larger than a threshold value based on the motion information,
the classification section excluding the pixel or the area for which it has been determined that the motion amount is larger than the threshold value from the target of the classification process.

11. The image processing device as defined in claim 10,
the classification section determining whether or not the pixel or the area agrees with characteristics of a normal structure to classify the pixel or the area as a normal part or a non-normal part, excluding the pixel or the area for which it has been determined that the motion amount is larger than the threshold value from the target of the classification process that classifies the pixel or the area as the normal part or the non-normal part, and classifying the pixel or the area for which it has been determined that the motion amount is larger than the threshold value as an unknown state that represents that it is unknown whether the pixel or the area should be classified as the normal part or the non-normal part.

12. The image processing device as defined in claim 9, further comprising:

a motion determination section that determines whether or not a motion amount of the object within a pixel or an area within the captured image is larger than a threshold value based on the motion information,
the classification section correcting a result of the classification process with respect to the pixel or the area for which it has been determined that the motion amount is larger than the threshold value.

13. The image processing device as defined in claim 12,
the classification section determining whether or not the pixel or the area agrees with characteristics of a normal structure to classify the pixel or the area as a normal part or a non-normal part, and correcting a classification result that represents the normal part or the non-normal part to an unknown state with respect to the pixel or the area for which it has been determined that the motion amount is larger than the threshold value, the unknown state representing that it is unknown whether the pixel or the area should be classified as the normal part or the non-normal part.

14. The image processing device as defined in claim 9,
the motion detection section converting the motion information detected based on the captured image into the motion information based on the object based on the distance information, and
the classification section controlling the target of the classification process corresponding to the motion information based on the object.

**15.** The image processing device as defined in claim 9, further comprising:

an enhancement processing section that performs the enhancement process on the captured image based on results of the classification process.

**16.** The image processing device as defined in claim 1 or 9, further comprising:

a known characteristic information acquisition section that acquires known characteristic information, the known characteristic information being information that represents known characteristics relating to a structure of the object,
the classification section including:

a surface shape calculation section that calculates surface shape information about the object based on the distance information and the known characteristic information; and
a classification processing section that generates a classification reference based on the surface shape information, and performs the classification process that utilizes the generated classification reference.

**17.** The image processing device as defined in claim 16,
the known characteristic information acquisition section acquiring a reference pattern that corresponds to the structure of the object in a given state as the known characteristic information, and
the classification processing section generating a corrected pattern as the classification reference, and performing the classification process using the generated classification reference, the corrected pattern being acquired by performing a deformation process based on the surface shape information on the reference pattern.

**18.** An endoscope apparatus comprising the image processing device as defined in any one of claims 1 to 15.

**19.** A program that causes a computer to perform steps of:

acquiring a captured image in time series, the captured image including an image of an object;
acquiring distance information based on a distance from an imaging section to the object when the imaging section captured the captured image;
detecting motion information about a local motion of the object based on the captured image acquired in time series;
performing a classification process that classifies a structure of the object based on the distance information; and
performing an enhancement process on the captured image based on results of the classification process, and controlling a target or an enhancement level of the enhancement process corresponding to the motion information about the local motion of the object.

**20.** A program that causes a computer to perform steps of:

acquiring a captured image in time series, the captured image including an image of an object;
acquiring distance information based on a distance from an imaging section to the object when the imaging section captured the captured image;
detecting motion information about a local motion of the object based on the captured image acquired in time series; and
performing a classification process that classifies a structure of the object based on the distance information, and controlling a target of the classification process corresponding to the motion information about the local motion of the object.

**21.** An image processing method comprising:

acquiring a captured image in time series, the captured image including an image of an object;
acquiring distance information based on a distance from an imaging section to the object when the imaging section captured the captured image;
detecting motion information about a local motion of the object based on the captured image acquired in time series;
performing a classification process that classifies a structure of the object based on the distance information; and
performing an enhancement process on the captured image based on results of the classification process, and controlling a target or an enhancement level of the enhancement process corresponding to the motion information about the local motion of the object.

22. An image processing method comprising:

acquiring a captured image in time series, the captured image including an image of an object;
acquiring distance information based on a distance from an imaging section to the object when the imaging section captured the captured image;
detecting motion information about a local motion of the object based on the captured image acquired in time series; and
performing a classification process that classifies a structure of the object based on the distance information, and controlling a target of the classification process corresponding to the motion information about the local motion of the object.

**Amended claims under Art. 19.1 PCT**

1. (Amended) An image processing device comprising:

an image acquisition section that acquires a captured image in time series, the captured image including an image of an object;
a distance information acquisition section that acquires distance information based on a distance from an imaging section to the object when the imaging section captured the captured image;
a motion detection section that detects motion information about a local motion of the object based on the captured image acquired in time series;
a classification section that performs a classification process that classifies a structure of the object based on the distance information; and
an enhancement processing section that performs an enhancement process on the captured image based on results of the classification process, and controls a target or an enhancement level of the enhancement process corresponding to the motion information about the local motion of the object,
the enhancement processing section excluding a pixel or an area within the captured image for which it has been determined that a motion amount of the object is larger than a threshold value from the target of the enhancement process based on a classification result based on the motion information, or decreasing the enhancement level of the enhancement process applied to the pixel or the area within the captured image as the motion amount of the object within the pixel or the area increases based on the motion information.

2. (Amended) The image processing device as defined in claim 1, further comprising:

a motion determination section that determines whether or not the motion amount within the pixel or the area is larger than the threshold value based on the motion information,
the enhancement processing section excluding the pixel or the area for which it has been determined that the motion amount is larger than the threshold value from the target of the enhancement process.

3. The image processing device as defined in claim 2,
the classification section determining whether or not the pixel or the area agrees with characteristics of a normal structure to classify the pixel or the area as a normal part or a non-normal part, and
the enhancement processing section excluding the pixel or the area for which it has been determined that the motion amount is larger than the threshold value from the target of the enhancement process based on a classification result that represents the normal part or the non-normal part.

4. deleted)

5. (Amended) The image processing device as defined in claim 1, further comprising:

a motion determination section that determines whether or not the motion amount of the object within the pixel or the area within the captured image is larger than the threshold value based on the motion information,
the classification section excluding the pixel or the area from a target of the classification process when the motion determination section has determined that the motion amount is larger than the threshold value.

**6.** The image processing device as defined in claim 1,
the motion detection section converting the motion information detected based on the captured image into the motion information based on the object based on the distance information, and
the enhancement processing section controlling the target or the enhancement level of the enhancement process corresponding to the motion information based on the object.

**7.** The image processing device as defined in claim 6,
the motion detection section including an imaging condition acquisition section that acquires an imaging condition employed when the captured image was captured, and
the motion detection section calculating the motion information based on the object based on the distance information and the imaging condition.

**8.** The image processing device as defined in claim 7,
the imaging condition being a magnification of an optical system of the imaging section that corresponds to the distance information, and
the motion detection section calculating the motion information based on the object by multiplying the motion information detected based on the captured image by the magnification.

**9.** An image processing device comprising:

an image acquisition section that acquires a captured image in time series, the captured image including an image of an object;
a distance information acquisition section that acquires distance information based on a distance from an imaging section to the object when the imaging section captured the captured image;
a motion detection section that detects motion information about a local motion of the object based on the captured image acquired in time series; and
a classification section that performs a classification process that classifies a structure of the object based on the distance information, and controls a target of the classification process corresponding to the motion information about the local motion of the object.

**10.** The image processing device as defined in claim 9, further comprising:

a motion determination section that determines whether or not a motion amount of the object within a pixel or an area within the captured image is larger than a threshold value based on the motion information,
the classification section excluding the pixel or the area for which it has been determined that the motion amount is larger than the threshold value from the target of the classification process.

**11.** The image processing device as defined in claim 10,
the classification section determining whether or not the pixel or the area agrees with characteristics of a normal structure to classify the pixel or the area as a normal part or a non-normal part, excluding the pixel or the area for which it has been determined that the motion amount is larger than the threshold value from the target of the classification process that classifies the pixel or the area as the normal part or the non-normal part, and classifying the pixel or the area for which it has been determined that the motion amount is larger than the threshold value as an unknown state that represents that it is unknown whether the pixel or the area should be classified as the normal part or the non-normal part.

**12.** The image processing device as defined in claim 9, further comprising:

a motion determination section that determines whether or not a motion amount of the object within a pixel or an area within the captured image is larger than a threshold value based on the motion information,
the classification section correcting a result of the classification process with respect to the pixel or the area for which it has been determined that the motion amount is larger than the threshold value.

**13.** The image processing device as defined in claim 12,
the classification section determining whether or not the pixel or the area agrees with characteristics of a normal structure to classify the pixel or the area as a normal part or a non-normal part, and correcting a classification result that represents the normal part or the non-normal part to an unknown state with respect to the pixel or the area for which it has been determined that the motion amount is larger than the threshold value, the unknown state representing that it is unknown whether the pixel or the area should be classified as the normal part or the non-normal part.

**14.** The image processing device as defined in claim 9,
the motion detection section converting the motion information detected based on the captured image into the motion information based on the object based on the distance information, and
the classification section controlling the target of the classification process corresponding to the motion information based on the object.

**15.** The image processing device as defined in claim 9, further comprising:

an enhancement processing section that performs the enhancement process on the captured image based on results of the classification process.

**16.** The image processing device as defined in claim 1 or 9, further comprising:

a known characteristic information acquisition section that acquires known characteristic information, the known characteristic information being information that represents known characteristics relating to a structure of the object,
the classification section including:

a surface shape calculation section that calculates surface shape information about the object based on the distance information and the known characteristic information; and
a classification processing section that generates a classification reference based on the surface shape information, and performs the classification process that utilizes the generated classification reference.

**17.** The image processing device as defined in claim 16,
the known characteristic information acquisition section acquiring a reference pattern that corresponds to the structure of the object in a given state as the known characteristic information, and
the classification processing section generating a corrected pattern as the classification reference, and performing the classification process using the generated classification reference, the corrected pattern being acquired by performing a deformation process based on the surface shape information on the reference pattern.

**18.** An endoscope apparatus comprising the image processing device as defined in any one of claims 1 to 15.

**19.** (Amended) A program that causes a computer to perform steps of:

acquiring a captured image in time series, the captured image including an image of an object;
acquiring distance information based on a distance from an imaging section to the object when the imaging section captured the captured image;
detecting motion information about a local motion of the object based on the captured image acquired in time series;
performing a classification process that classifies a structure of the object based on the distance information;
performing an enhancement process on the captured image based on results of the classification process, and controlling a target or an enhancement level of the enhancement process corresponding to the motion information about the local motion of the object;
excluding a pixel or an area within the captured image for which it has been determined that a motion amount of the object is larger than a threshold value from the target of the enhancement process based on a classification result based on the motion information when controlling the target of the enhancement process; and
decreasing the enhancement level of the enhancement process applied to the pixel or the area within the captured image as the motion amount of the object within the pixel or the area increases based on the motion information when controlling the enhancement level.

**20.** A program that causes a computer to perform steps of:

acquiring a captured image in time series, the captured image including an image of an object;
acquiring distance information based on a distance from an imaging section to the object when the imaging section captured the captured image;
detecting motion information about a local motion of the object based on the captured image acquired in time series; and

performing a classification process that classifies a structure of the object based on the distance information, and controlling a target of the classification process corresponding to the motion information about the local motion of the object.

**21.** (Amended) An image processing method comprising:

acquiring a captured image in time series, the captured image including an image of an object;

acquiring distance information based on a distance from an imaging section to the object when the imaging section captured the captured image;

detecting motion information about a local motion of the object based on the captured image acquired in time series;

performing a classification process that classifies a structure of the object based on the distance information;

performing an enhancement process on the captured image based on results of the classification process, and controlling a target or an enhancement level of the enhancement process corresponding to the motion information about the local motion of the object;

excluding a pixel or an area within the captured image for which it has been determined that a motion amount of the object is larger than a threshold value from the target of the enhancement process based on a classification result based on the motion information when controlling the target of the enhancement process; and

decreasing the enhancement level of the enhancement process applied to the pixel or the area within the captured image as the motion amount of the object within the pixel or the area increases based on the motion information when controlling the enhancement level.

**22.** An image processing method comprising:

acquiring a captured image in time series, the captured image including an image of an object;

acquiring distance information based on a distance from an imaging section to the object when the imaging section captured the captured image;

detecting motion information about a local motion of the object based on the captured image acquired in time series; and

performing a classification process that classifies a structure of the object based on the distance information, and controlling a target of the classification process corresponding to the motion information about the local motion of the object.

# FIG. 1A

# FIG. 1B

# FIG. 2A

# FIG. 2B

# FIG. 3

| 305 | 340 | 310 |
|---|---|---|
| IMAGE ACQUISITION SECTION | DISTANCE INFORMATION ACQUISITION SECTION | CLASSI-FICATION SECTION |
| | MOTION DETECTION SECTION | ENHANCEMENT PROCESSING SECTION |
| | 380 | 330 |

IMAGE PROCESSING DEVICE

# FIG. 4

| 305 | 340 | 310 |
|---|---|---|
| IMAGE ACQUISITION SECTION | DISTANCE INFORMATION ACQUISITION SECTION | CLASSI-FICATION SECTION |
| | MOTION DETECTION SECTION | |
| | 380 | |

IMAGE PROCESSING DEVICE

# FIG. 5

FIG. 6

FIG. 7

FIG. 8

FIG. 9

START

INPUT VIDEO HEADER — S11

INPUT IMAGE SIGNALS — S12

CALCULATE DISTANCE MAP — S13

CALCULATE
THREE-DIMENSIONAL SHAPE — S14

CORRECT
CLASSIFICATION REFERENCE — S15

IMAGE CONSTRUCTION — S16

DETECT MOTION — S17

STORE IMAGE IN MEMORY — S18

CLASSIFICATION — S19

ENHANCEMENT PROCESS — S20

OUTPUT IMAGE SIGNALS — S21

S22

NO    FINAL IMAGE?

YES

END

FIG. 10

EP 2 979 607 A1

# FIG. 11

FIG. 12

EP 2 979 607 A1

FIG. 13

# FIG. 14A

# FIG. 14B

(MOTION WITHIN IMAGE
THAT CORRESPONDS
TO NEAR POINT)
MD1    40    50

MD2
(MOTION WITHIN IMAGE
THAT CORRESPONDS
TO MIDDLE/FAR POINT)

# FIG. 14C

(EXCLUDED FROM
DETECTION RANGE
SINCE MOTION IS LARGE)
GR3

GR1
(NOT EXCLUDED FROM
DETECTION RANGE
SINCE MOTION IS SMALL)

# FIG. 15

| DISTANCE d FROM IMAGE SENSOR[mm] | MAGNIFICATION K(d) OF OPTICAL SYSTEM |
|:---:|:---:|
| 0.1 | K(0.1) |
| 0.2 | K(0.2) |
| ⋮ | ⋮ |
| D | K(D) |

# FIG. 16

EP 2 979 607 A1

310

CLASSIFICATION SECTION

345

KNOWN
CHARACTERISTIC
INFORMATION
ACQUISITION
SECTION

340

DISTANCE
INFORMATION
ACQUISITION
SECTION

360

SURFACE SHAPE
CALCULATION
SECTION

350

CLASSIFICATION
PROCESSING
SECTION

330

ENHANCEMENT
PROCESSING
SECTION

IMAGE
CONSTRUCTION
SECTION

320

# FIG. 17A

CLOSING PROCESS

DISTANCE

# FIG. 17B

CALCULATE NORMAL VECTOR WITH RESPECT
TO RESULTS OF CLOSING PROCESS

# FIG. 18A

BASIC PIT

# FIG. 18B

CORRECTED PIT

# FIG. 19

EP 2 979 607 A1

# FIG. 20

KNOWN CHARACTERISTIC INFORMATION ACQUISITION SECTION — 345

SURFACE SHAPE CALCULATION SECTION — 350

DISTANCE INFORMATION ACQUISITION SECTION — 340

CLASSIFICATION PROCESSING SECTION — 360

CLASSIFICATION REFERENCE DATA STORAGE SECTION — 361

PROJECTIVE TRANSFORMATION SECTION — 362

SEARCH AREA SIZE SETTING SECTION — 363

SIMILARITY CALCULATION SECTION — 364

AREA SETTING SECTION — 365

ENHANCEMENT PROCESSING SECTION — 330

IMAGE CONSTRUCTION SECTION — 320

EP 2 979 607 A1

# FIG. 21A

PROCESSING
TARGET POSITION

CAPTURED IMAGE

# FIG. 21B

CORRECTED PATTERN

# FIG. 21C

PROCESSING
TARGET POSITION

SEARCH AREA

# FIG. 21D

SEARCH AREA

# FIG. 21E

SIMILARITY
VALUE PEAK

AREA THAT
AGREES WITH
CLASSIFICATION
REFERENCE

SEARCH AREA

# FIG. 21F

SIMILARITY
VALUE PEAK

AREA THAT
AGREES WITH
CLASSIFICATION
REFERENCE

SEARCH AREA

# FIG. 22

KNOWN CHARACTERISTIC INFORMATION ACQUISITION SECTION — 345

SURFACE SHAPE CALCULATION SECTION — 350

DISTANCE INFORMATION ACQUISITION SECTION — 340

CLASSIFICATION PROCESSING SECTION — 360

CLASSIFICATION REFERENCE DATA STORAGE SECTION — 361

SECOND CLASSIFICATION REFERENCE DATA GENERATION SECTION — 366

PROJECTIVE TRANSFORMATION SECTION — 362

SIMILARITY CALCULATION SECTION — 364

AREA SETTING SECTION — 365

SEARCH AREA SIZE SETTING SECTION — 363

ENHANCEMENT PROCESSING SECTION — 330

IMAGE CONSTRUCTION SECTION — 320

# FIG. 23

| CLASS | PIT |
|---|---|
| TYPE I | TYPE A |
| TYPE II | TYPE B |
| | TYPE C |
| TYPE III | TYPE D |
| | TYPE E |
| | TYPE F |
| ... | ... |

FIG. 24A

I

FIG. 24B

II

FIG. 24C

IIIs

FIG. 24D

IIIL

FIG. 24E

IV

FIG. 24F

V

## INTERNATIONAL SEARCH REPORT

| International application No. |
| --- |
| PCT/JP2013/075629 |

**A. CLASSIFICATION OF SUBJECT MATTER**
*A61B1/00*(2006.01)i, *A61B1/04*(2006.01)i, *G02B23/24*(2006.01)i, *G06T1/00* (2006.01)i

According to International Patent Classification (IPC) or to both national classification and IPC

**B. FIELDS SEARCHED**

Minimum documentation searched (classification system followed by classification symbols)
A61B1/00, A61B1/04, G02B23/24, G06T1/00

Documentation searched other than minimum documentation to the extent that such documents are included in the fields searched
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y<br>A | JP 2008-278963 A (Olympus Corp.),<br>20 November 2008 (20.11.2008),<br>paragraphs [0012] to [0042]; fig. 1 to 7<br>& US 2008/0279431 A1 & EP 2149330 A1<br>& WO 2008/139812 A1 | 1,18,19<br>2-17,20 |
| Y<br>A | JP 2009-71873 A (Sharp Corp.),<br>02 April 2009 (02.04.2009),<br>abstract<br>& JP 2008-295022 A & JP 2008-295023 A<br>& JP 2011-120270 A & JP 2012-231471 A<br>& JP 4139430 B & US 2010/0091185 A1<br>& US 2010/0110300 A1 & EP 2141910 A1<br>& EP 2144434 A1 & WO 2008/136286 A1<br>& WO 2008/136289 A1 & CN 101663885 A<br>& CN 101663886 A & RU 2009135233 A<br>& CN 102394051 A | 1,18,19<br>2-17,20 |

☒ Further documents are listed in the continuation of Box C. ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 19 December, 2013 (19.12.13) | 07 January, 2014 (07.01.14) |

| Name and mailing address of the ISA/<br>Japanese Patent Office | Authorized officer |
| --- | --- |
| Facsimile No. | Telephone No. |

Form PCT/ISA/210 (second sheet) (July 2009)

**INTERNATIONAL SEARCH REPORT**

| International application No. |
| --- |
| PCT/JP2013/075629 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y<br>A | JP 2012-10214 A  (Panasonic Corp.),<br>12 January 2012 (12.01.2012),<br>abstract; paragraph [0052]<br>(Family: none) | 1,18,19<br>2-17,20 |
| A | JP 2010-68865 A  (Fujifilm Corp.),<br>02 April 2010 (02.04.2010),<br>abstract<br>& US 2010/0069747 A1     & EP 2163191 A1 | 1-20 |

Form PCT/ISA/210 (continuation of second sheet) (July 2009)

## INTERNATIONAL SEARCH REPORT

| International application No. |
|---|
| PCT/JP2013/075629 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 21,22
   because they relate to subject matter not required to be searched by this Authority, namely:
   The invention in claims 21 and 22 falls under the category of methods for treatment of the body of a human being by surgery, because the invention is conducted during the period when an endoscope is used in the human body.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**EP 2 979 607 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010068865 A